# EUROPEAN PATENT SPECIFICATION

(11) **EP 3 262 164 B1**
(45) Date of publication and mention of the grant of the patent: **12.02.2020**
(21) Application number: 16707039.0
(22) Date of filing: 24.02.2016
(51) Int. Cl.: C12N 9/42, C12P 19/02, C12N 9/24

(54) **HEMICELLULOSE-DEGRADING COMPOSITIONS AND USES THEREOF**
HEMICELLULOSEABBAUENDE ZUSAMMENSETZUNGEN UND VERWENDUNGEN DAVON
COMPOSITIONS DÉGRADANT L'HÉMICELLULOSE ET UTILISATIONS DE CELLES-CI

(30) Priority: 25.02.2015 EP 15305289
(43) Date of publication of application: 03.01.2018
(73) Proprietor: Carbios, 63360 Saint-Beauzire (FR)
(72) Inventor: O'DONOHUE, Michael, 31500 Toulouse (FR); DUMON, Claire, 31320 Auzeville-Tolosane (FR); ALVIRA, Pablo, 31003 Pamplona (ES)
(74) Representative: Cabinet Becker et Associés
(86) International application number: PCT/EP2016/053822
(87) International publication number: WO 2016/135173

(56) References cited:
- WO-A2-2009/108941
- WO-A2-2010/117843
- GÉRALDINE BASTIEN ET AL: "Mining for hemicellulases in the fungus-growing termite Pseudacanthotermes militaris using functional metagenomics", BIOTECHNOLOGY FOR BIOFUELS, BIOMED CENTRAL LTD, GB, vol. 6, no. 1, 14 May 2013 (2013-05-14), page 78, XP021151520, ISSN: 1754-6834, DOI: 10.1186/1754-6834-6-78 -& "UNIPROT:S0DEW6", , 18 September 2013 (2013-09-18), XP055200697, Retrieved from the Internet: URL:http://ibis/exam/dbfetch.jsp?id=UNIPRO T:S0DEW6 [retrieved on 2015-07-07] -& Anonymous: "UNIPROT:S0DFK9", , 18 September 2013 (2013-09-18), XP055201376, Retrieved from the Internet: URL:http://ibis/exam/dbfetch.jsp?id=UNIPRO T:S0DFK9 [retrieved on 2015-07-09]
- JOÃO PAULO L FRANCO CAIRO ET AL: "Functional characterization and target discovery of glycoside hydrolases from the digestome of the lower termite Coptotermes gestroi", BIOTECHNOLOGY FOR BIOFUELS, BIOMED CENTRAL LTD, GB, vol. 4, no. 1, 14 November 2011 (2011-11-14), page 50, XP021131897, ISSN: 1754-6834, DOI: 10.1186/1754-6834-4-50
- JINFENG NI ET AL: "Lignocellulose-degrading enzymes from termites and their symbiotic microbiota", BIOTECHNOLOGY ADVANCES, vol. 31, no. 6, 1 November 2013 (2013-11-01), pages 838-850, XP055200669, ISSN: 0734-9750, DOI: 10.1016/j.biotechadv.2013.04.005

## Description

The present invention relates to new enzymatic compositions and the uses thereof to hydrolyze hemicellulose. More specifically, the invention relates to improved enzymatic compositions containing a new polypeptide having arabinofuranosidase and β-xylosidase activity, and the uses thereof for modifying a hemicellulose-containing biomass. The invention further relates to methods for producing fermentable sugars from biomass using such enzymatic compositions.

### Background of the invention

Cellulose and hemicellulose are the first and second most abundant polysaccharides in nature. Cellulose represents from 30 to 60% and hemicellulose represents about 20-40% of lignocellulosic biomass such as in corn fiber, corn stover, wheat straw, rice straw, and sugarcane bagasse. Hemicelluloses are heterogeneous polymers of pentoses (xylose, arabinose), hexoses (mannose, glucose, galactose), and acid sugars. For instance, hardwood hemicelluloses contain mostly xylans, whereas softwood hemicelluloses contain mostly glucomannans.
Biomass wastes and byproducts containing hemicellulose are generated by numerous manufacturing processes, and food and agricultural processing. A common example of such biomass material is the waste and byproducts from grain processing such as the stalks and leaves of the grain plant. For instance, wheat bran is a large volume biomass material that includes up to 40% weight hemicellulose. Disposal of such biomass material is becoming an increasing problem and the conversion of biomass material to higher value materials has considerable interest.
Additionally, fermentable sugars are increasingly used to produce alcohol (e.g., ethanol) as an alternative to liquid fuels, plastics, polymers and other bio-based materials such as lactic acid. The demand for abundant low cost fermentable sugars, which can be used in lieu of petroleum-based fuel feedstock, is growing rapidly. Typically, the enzymatic conversion of hemicellulose to fermentable sugars, e.g., glucose, xylose, arabinose, galactose, mannose, and/or other hexose (C6) and pentose (C5) sugars, leads to a useful and abundant renewable source of raw material for a variety of useful chemicals and biochemicals.
Three major approaches have been developed to convert hemicellulosic biomass (i.e. biomass containing a high content of hemicellulose, such as at least 10% by weight of hemicellulose) into fermentable sugars.

First is chemical hydrolysis, wherein hemicellulose is generally separated in the first step from the lignocellulose composite material by the action of an acid or alkali. In a second step a higher temperature acid hydrolysis process hydrolyzes the plant material cellulose. Despite its efficiency to produce C6 sugars, the acid process also produces other by-products that interfere with the use of the sugars in downstream processes like fermentation to useful products like lactic acid, alcohols, organic acids etc. Furthermore, in order to reduce effluent and pollution problems it is required to recover or neutralize the acids at the end of the process.
The second approach is thermal hydrolysis. Said thermal processes, such as Steam Explosion processes, are cleaner since they do not make use of any chemicals. However, these processes lead to low sugar yields, formation of unwanted by-products that may inhibit downstream processes, and are energy intensive.
The third approach consists on enzymatic hydrolysis, optionally preceded by pretreatment steps. Enzymatic processes use enzymatic compositions for hydrolyzing hemicellulose. These processes are much cleaner and low energy consumers for hemicellulose hydrolysis and finally provide better quality fermentable sugars in higher yields. However, the hydrolytic efficiency of an enzymatic composition can depend on a multitude of factors, e.g., hydrolytic properties of individual enzymes, the synergies among them, their ratio in the mixture, etc. The hydrolytic efficiency of enzymatic compositions is one of the major factors that restrict these enzymatic processes.

Despite substantial progress and huge interest in the field, there is still a need in the art to identify enzymes and enzymatic mixtures or blends that are suitable for converting hemicellulose contained in a biomass into fermentable sugars with sufficient or improved efficacy, improved fermentable sugar yields, and/or improved capacity to act on a greater variety of biomasses containing hemicellulose.

### Summary of the invention

The present invention provides novel compositions having improved hemicellulolytic activity and the uses thereof to transform hemicellulose-containing biomass. In particular, the inventors have identified an enzyme of the Glycoside Hydrolase family 3 (GH3) which exhibits remarkable dual arabinofuranosidase and β-xylosidase activity. The inventors have further shown that this enzyme may be efficiently used in an enzymatic mixture or blend, such as in combination with a xylanase, to optimize hydrolysis of hemicellulose, particularly arabinoxylan contained in hemicellulose.

It is therefore an object of the present invention to provide a composition comprising at least (i) a polypeptide having at least 55%, 60%, 65%, 70%, 75%, 80%, 85%, 90%, 95%, 99% or 100% identity to the full length amino acid sequence set forth in SEQ ID N°2, and (ii) a xylanase.

Advantageously, the composition further comprises at least one polypeptide selected from the group consisting of a β-xylosidase, an arabinanase, a glucanase or an esterase.

The invention further relates to the use of such composition for modifying a hemicellulose-containing biomass.

The invention also relates to the use of a polypeptide having at least 55%, 60%, 65%, 70%, 75%, 80%, 85%, 90%, 95%, 99% or 100% identity to the full length amino acid sequence set forth in SEQ ID NO: 2, or of a microorganism expressing said polypeptide, for modifying a hemicellulose-containing biomass, and optionally producing fermentable sugars from the hemicellulose-containing biomass, more preferably xylose and arabinose.

It is another object of the invention to provide a method for modifying a hemicellulose-containing biomass, comprising:
a) contacting such a biomass with a polypeptide having at least 55%, 60%, 65%, 70%, 75%, 80%, 85%, 90%, 95%, 99% or 100% identity to the full length amino acid sequence set forth in SEQ ID NO: 2, or with a microorganism expressing said polypeptide;
b) optionally recovering fermentable sugars from the modified biomass of step a), and
c) optionally fermenting the fermentable sugars of step a) or b).
In a particular embodiment, the biomass is contacted in step a) with at least one further polypeptide selected from the group consisting of a xylanase, a β-xylosidase, an arabinanase, a glucanase, and/or an esterase.

It is a further object of the invention to provide a method for modifying a hemicellulose-containing biomass, comprising contacting the biomass with a composition as defined above.

The invention also discloses a host cell, such as a lactic acid bacterium, comprising at least a nucleotide sequence encoding a polypeptide having at least 55%, 60%, 65%, 70%, 75%, 80%, 85%, 90%, 95%, 99% or 100% identity to the full length amino acid sequence set forth in SEQ ID N°2.

The invention further relates to a kit for hydrolyzing hemicellulose, comprising at least
- a polypeptide having at least 55%, 60%, 65%,70%, 75%, 80%, 85%, 90%, 95%, 99% or 100% identity to the full length amino acid sequence set forth in SEQ ID N°2; and
- a xylanase, preferably a xylanase of Glycoside Hydrolase family 11 (GH11) or 10 (GH10), with preferably at least 55%, 60%, 65%, 70%, 75%, 80%, 85%, 90%, 95%, 99% or 100% identity to the full length amino acid sequence set forth in SEQ ID N°4 or 6 respectively.

### Legend to the figures

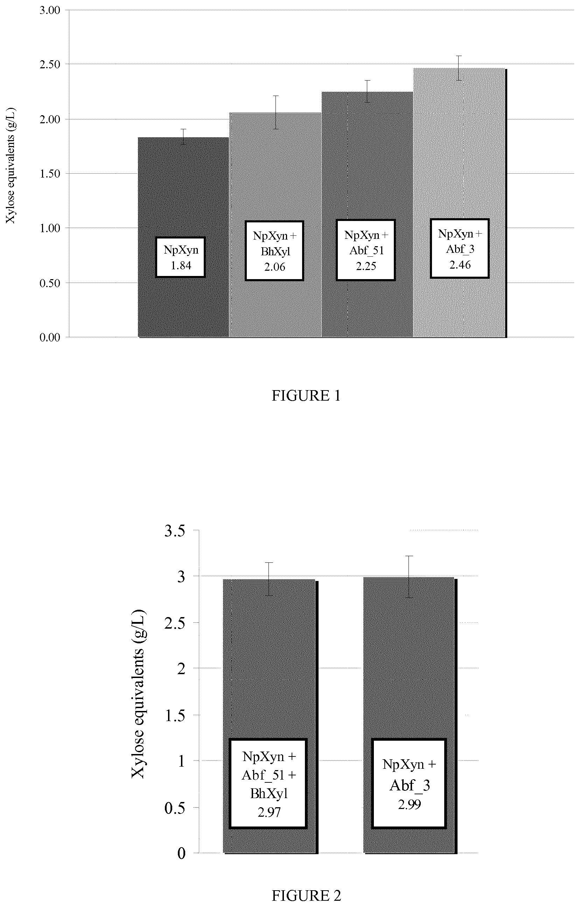

**Figure 1** shows the enzymatic hydrolysis of wheat bran using different enzymatic cocktails. Assays were performed at 2% substrate concentration, pH6, 30 °C and for 24h. Reducing sugars (xylose equivalents) were determined by DNS method;
**Figure 2** shows a comparison of the effects of an enzymatic cocktail of the invention comprising arabinofuranosidase Abf_3 and a xylanase, with an enzymatic cocktail comprising an arabinofuranosidase of the prior art (Abf_51), a β-xylosidase and a xylanase on the enzymatic hydrolysis of wheat bran. Enzymatic hydrolysis assays were performed using 2% wheat bran at pH6, 30 °C during 24h. Reducing sugars (xylose equivalents) were determined by DNS method;

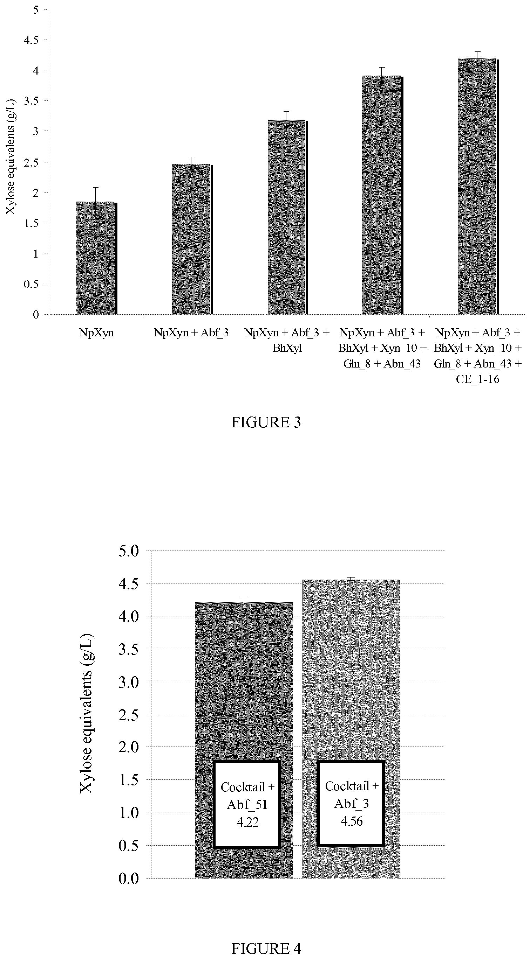

**Figure 3** shows the positive effects of additional enzymatic activities included in the enzymatic cocktail of the invention, on the enzymatic hydrolysis of wheat bran. Assays were performed at 2% substrate concentration, pH6, 30 °C during 24h. Reducing sugars (xylose equivalents) were determined by DNS method;
**Figure 4** shows a comparison of the effects of an arabinofuranosidase of the prior art (Abf_51) to the effects of the arabinofuranosidase of the invention (Abf_3) on the enzymatic hydrolysis of wheat bran. Assays were performed at 2% substrate concentration, pH6, 30 °C during 24h. Reducing sugars (xylose equivalents) were determined by DNS method;

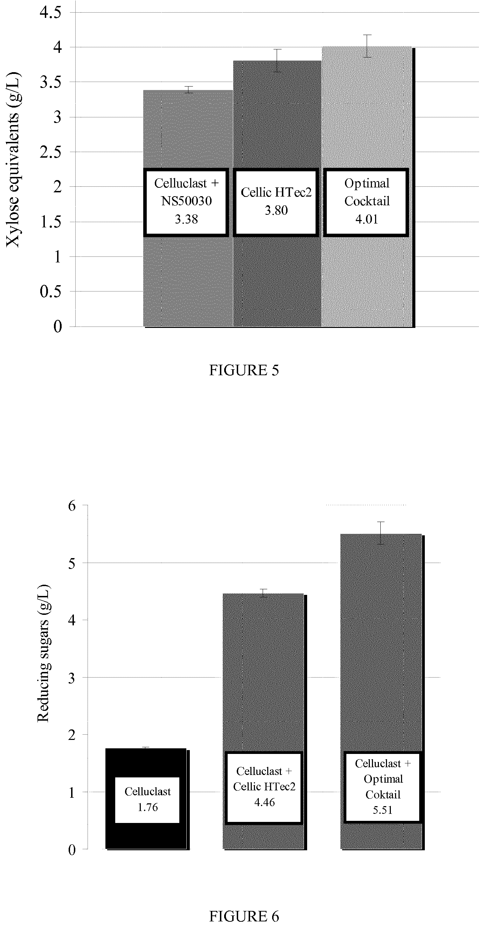

**Figure 5** shows a comparison of the effects of different enzymatic compositions commercially available to the effects of an enzymatic composition of the invention on the enzymatic hydrolysis of wheat bran. Assays were performed at 2% substrate concentration, pH6, 30 °C during 24h. Reducing sugars (xylose equivalents) were determined by DNS method;
**Figure 6** shows the effect of a cellulase supplementation in different enzymatic compositions on the enzymatic hydrolysis of wheat bran. Assays were performed at 2% substrate concentration, pH6, 30 °C during 24h. Reducing sugars were determined by DNS method;

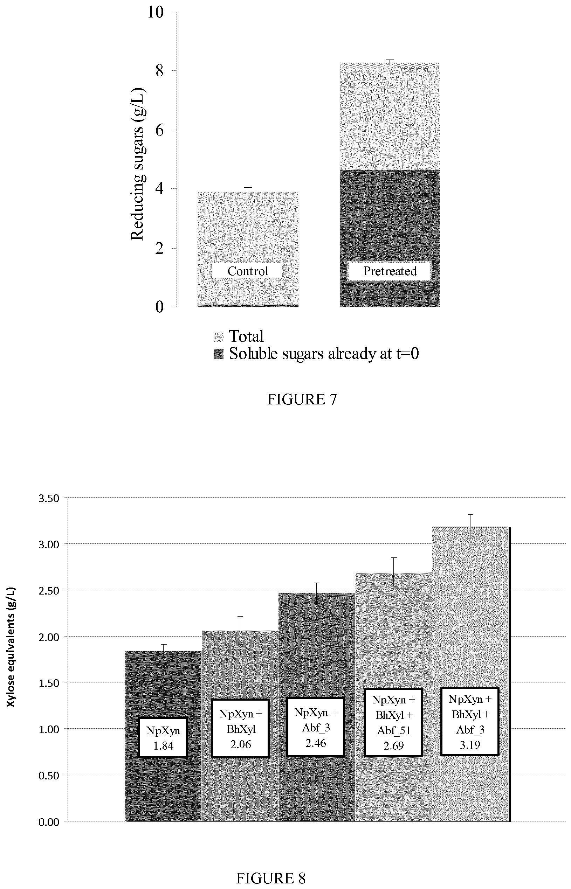

**Figure 7** shows a comparison of the effects of an enzymatic composition of the invention to the effects of a control composition on the enzymatic hydrolysis of pretreated wheat bran. Wheat bran was submitted to a physicochemical treatment. Assays were performed at 2% substrate concentration, pH6, 30 °C during 24h. Reducing sugars (xylose equivalents) were determined by DNS method;
**Figure 8** shows the enzymatic hydrolysis of wheat bran using different enzymatic mixture; Assays were performed at 2% substrate concentration, pH6, 30 °C during 24h. Reducing sugars (xylose equivalents) were determined by DNS method.

### Detailed description of the invention

The following is a description of the present invention, including preferred embodiments thereof given in general terms. The present invention is further exemplified in the disclosure given under the heading "Examples" herein below, which provides experimental data supporting the invention and means of performing the invention.

### Definitions

The present disclosure will be best understood by reference to the following definitions.

In the context of the invention, the terms *"composition", "blend" and "mixture"* are used interchangeably and encompass all kind of compositions comprising polypeptide(s) having an enzymatic activity. For instance, the mixture may comprise polypeptides and/or host cells encoding polypeptide(s) of interest or extract thereof, and optionally excipients and/or fermenting microorganism(s). The mixture may further comprise a biomass, optionally at least partially hydrolyzed. In a composition of the invention, the various constituents are typically in admixture. They may be in solid or liquid state, such as a solution, suspension, paste, gel, lyophilisate or frozen preparation.

The term *"biomass",* in the context of the invention, designates any hemicellulose-containing materials, particularly any hemicellulose-containing vegetal material. Typically, biomass designates any plant biomass, particularly any unprocessed or processed material of vegetal origin. Examples of biomass include, without limitation, plant material, forestry products, including mature trees unsuitable for lumber or paper production, agricultural products, such as grasses, crops and animal manure, and aquatic products, such as algae and seaweed. Plant material and agricultural products include, but are not limited to, Miscanthus, energy grass, elephant grass, switchgrass, cord grass, rye grass, rye bran, reed canary grass, common reed, wheat straw, wheat bran, barley, barley straw, barley bran, canola straw, oats, oat straw, oat bran, corn stover, soybean stover, oat hulls, oat spelt, sorghum, rice hulls, rice bran, sugarcane bagasse, pressed sugarcane stalk, corn fiber, flax, wheat, linseed, citrus pulp, cottonseed, groundnut, rapeseed, sunflower, peas, lupines, palm kernel, coconut, konjac, locust bean gum, gum guar, soy beans, Distillers Dried Grains with Solubles (DDGS), wet or dried distillers grain, Blue Stem, corncobs, energy cane, cassava peels, cocoa pods, spent grain, vinasse, wood pulp fiber, sawdust, sugar beet pulp, locust bean pulp or other vegetable or fruit pomaces, pine, conifer softwood, eucalyptus, birchwood, willow, aspen, poplar wood, hybrid poplar, hardwood, softwood, short-rotation woody crop, crop residue, yard waste, or any combination thereof (see for instance Sun et al., Bioresource Technology 83 (2002) 1-11). Generally speaking, a hemicellulose-containing material or biomass contains at least one polysaccharide selected from xylan and heteroxylan, such as glucuronoxylan, arabinoxylan, glucomannan or xyloglucan. Preferred biomasses contain at least 10 % by weight hemicellulose, more preferably at least 20 % by weight. Advantageously, the biomass contains at least 30% by weight of xylan and/or heteroxylan.
Preferred is a biomass obtained from cereal grain, e.g. such as milled grain or byproducts from processing of cereal grain. Preferred cereal grain is a cereal grain selected from the group consisting of corn (maize), wheat, barley, oat, rice, sorghum and millet.

*"Modifying"* a biomass, or biomass feedstock, within the context of the present invention includes any modification thereof, including transformation, degradation, hydrolysis, conversion or processing of a biomass. The term *"modifying"* a biomass typically encompasses any modification of the biomass that results in the production of fermentable sugars, monomeric sugars, metabolites, biofuels, feeds, foods and/or chemicals, or any other useful product. Modification also typically encompasses the hydrolysis or modification of biological polymers of the biomass, such as hemicellulose.

The term *"fermentable sugars"* designates, without limitation, carbohydrates having a basic composition (CH₂0)n. Based on the number of carbons (e.g., 3, 4, 5, or 6), the monosaccharide is a triose, (i.e., glyceraldehyde), tetrose, pentose (i.e., xylose), hexose (i.e., glucose), etc.

Herein, the terms *"peptide", "polypeptide"* and *"protein"* are employed interchangeably and refer to a chain of amino acids linked by peptide bonds, regardless of the number of amino acids forming said chain. The amino acids are herein represented by their one-letter or three-letter code according to the following nomenclature: A: alanine (Ala); C: cysteine (Cys); D: aspartic acid (Asp); E: glutamic acid (Glu); F: phenylalanine (Phe); G: glycine (Gly); H: histidine (His); I: isoleucine (Ile); K: lysine (Lys); L: leucine (Leu); M: methionine (Met); N: asparagine (Asn); P: proline (Pro); Q: glutamine (Gln); R: arginine (Arg); S: serine (Ser); T: threonine (Thr); V: valine (Val); W: tryptophan (Trp) and Y: tyrosine (Tyr).

As used herein, *"enzyme"* designates a polypeptide having catalytic or enzymatic activity.

As used herein, the term *"sequence identity"* or *"identity"* refers to the number (%) of matches (identical amino acid residues) in positions from an alignment of two polypeptide sequences. The sequence identity is determined by comparing the sequences when aligned so as to maximize overlap and identity while minimizing sequence gaps. In particular, sequence identity may be determined using any of a number of mathematical global or local alignment algorithms, depending on the length of the two sequences. Sequences of similar lengths are preferably aligned using a global alignment algorithms (e.g. Needleman and Wunsch algorithm; Needleman and Wunsch, 1970) which aligns the sequences optimally over the entire length, while sequences of substantially different lengths are preferably aligned using a local alignment algorithm (e.g. Smith and Waterman algorithm (Smith and Waterman, 1981) or Altschul algorithm (Altschul et al., 1997; Altschul et al., 2005)). Alignment for purposes of determining percent amino acid sequence identity can be achieved in various ways that are within the skill in the art, for instance, using publicly available computer software available on internet web sites such as http://blast.ncbi.nlm.nih.gov/ or http://www.ebi.ac.uk/Tools/emboss/). Those skilled in the art can determine appropriate parameters for measuring alignment, including any algorithms needed to achieve maximal alignment over the full length of the sequences being compared. For purposes herein, % amino acid sequence identity values refers to values generated using the pair wise sequence alignment program EMBOSS Needle that creates an optimal global alignment of two sequences using the Needleman-Wunsch algorithm, wherein all search parameters are set to default values, i.e. Scoring matrix = BLOSUM62, Gap open = 10, Gap extend = 0.5, End gap penalty = false, End gap open = 10 and End gap extend = 0.5.

The term *"expression",* as used herein, refers to any step involved in the production of a polypeptide including, but being not limited to, transcription, post-transcriptional modification, translation, post-translational modification, and secretion.

### Enzymes and compositions

The present invention is based in part on the discovery of certain novel enzymes having hemicellulolytic activities and, more particularly, of a new polypeptide exhibiting both arabinofuranosidase and β-xylosidase activity. The invention is also based on the design of novel compositions comprising particular blends or weight ratios of polypeptides having hemicellulolytic activities, allowing efficient hydrolysis of hemicellulosic materials. In the context of the invention, hemicellulolytic activities include, without limitation, arabinofuranosidase, xylanase, xylosidase, glucanase, pectinase such as arabinanase, and esterase activities.

In a first aspect, the invention thus relates to a polypeptide exhibiting both an arabinofuranosidase activity and a β-xylosidase activity and having at least 55%, 60%, 65%, 70%, 75%, 80%, 85%, 90%, 95%, 99% or 100% identity to the full length amino acid sequence set forth in SEQ ID N°2.

The invention further relates to a composition comprising (i) a polypeptide having at least 55%, 60%, 65%, 70%, 75%, 80%, 85%, 90%, 95%, 99% or 100% identity to the full length amino acid sequence set forth in SEQ ID N°2, herein after polypeptide Pax, and (ii) a xylanase.

According to the invention, the polypeptide Pax exhibits both an arabinofuranosidase activity and a β-xylosidase activity. Accordingly, the polypeptide Pax allows an efficient hydrolysis of arabinoxylan contained into hemicellulose containing biomass. It is important to note that the hydrolysis of arabinoxylan is a major prerequisite for improved hydrolysis of cereal hemicellulose. Indeed, arabinoxylan is part of the water soluble and insoluble fiber present in cereals, in particular in the cell walls. The use of the polypeptide Pax in a hemicellulolytic composition favors the degradation of cereal hydrolysis such as bran, wheat, etc.

The inventors have discovered that the newly identified polypeptide of SEQ ID N°2, herein after Abf_3, belongs to the Glycoside Hydrolase family 3 ("GH3"). The Glycoside Hydrolase family 3 typically comprises broad substrate specificity. They remove single glycosyl residues from the non-reducing ends of their substrates. Catalysis occurs via a classical Koshland double-displacement mechanism with the anomeric configuration of the released glycose being retained. GH3 can be multi modular proteins with a catalytic module a diversity of 3D-structures. The first GH3 structure described is composed of two domain (α/β)₈ barrel and (α/β)₆ sandwich (Varghese et al, Structure, 1999 Feb 15;7(2):179-90). GH3 enzymes may be found in every kingdom, including eukaryote, prokaryote, microbial, fungal and algae. For instance, the newly discovered enzyme of SEQ ID N°2 has been extracted from the Microbial community of insect's digestive tract.

As used herein, "Glycoside hydrolase" (GH), and "Carbohydrate Esterase" (CE) refer to two classes of carbohydrate-active enzymes distinguishable by the type of reaction catalyzed as defined by the CAZy database (Coutinho, P.M. & Henrissat, B. (1999) CAZy - Carbohydrate-Active Enzymes server at URL: http://afmb.cnrs-mrs.fr/~cazy/CAZY/index.html or alternatively Coutinho, P.M. & Henrissat, B. 1999; The modular structure of cellulases and other carbohydrate-active enzymes: an integrated database approach. In "Genetics, Biochemistry and Ecology of Cellulose Degradation"., K. Ohmiya, K. Hayashi, K. Sakka, Y. Kobayashi, S. Karita and T. Kimura eds., Uni Publishers Co., Tokyo, pp. 15-23, and Bourne, Y. & Henrissat, B. 2001; Glycoside hydrolases and glycosyltransferases: families and functional modules, Current Opinion in Structural Biology 11 :593-600). More precisely, GH's are a group of enzymes that hydrolyze the glycosidic bond between two carbohydrates, or between a carbohydrate and a non-carbohydrate moiety. The CAZy classification system has grouped GHs according to sequence similarity into about 133 different families.

According to the invention, the composition further comprises at least a xylanase. The combined use of the polypeptide Pax and a xylanase ensures breaking down hemicellulose.

In a particular embodiment, the composition comprises a xylanase, preferably a xylanase belonging to the Glycoside Hydrolase family 11 (GH11). Advantageously, the xylanase of has at least 55%, 60%, 65%, 70%, 75%, 80%, 85%, 90%, 95%, 99% or 100% identity to the full length amino acid sequence set forth in SEQ ID N°4 (herein after Xyn_11 or *Np*X*yn*).

Glycoside hydrolase family 11 ("GH11") enzymes have a β-jelly roll structure. They hydrolyze internal glycosidic bonds found in xylose-based polymers and are thus known as endo-acting enzymes that operate via a retaining mechanism that uses double displacement reaction with a glutamate residue acting as nucleophile and a glutamate residue being identified as acid/base residue. Several other residues spread throughout the +1 and -2 subsites may contribute to stabilizing the xylose units in the substrate neighboring the pair of xylose monomers that are cleaved by hydrolysis. GH11 enzymes may be found in every kingdom, including eukaryote, prokaryote, microbial, fungal and algae. In particular embodiments, the GH11 xylanase is extracted from the Microbial community of insect's digestive tract.

Alternatively, or in addition, the composition comprises a xylanase belonging to the Glycoside Hydrolase family 10 (GH10). Advantageously, the xylanase has at least 55%, 60%, 65%, 70%, 75%, 80%, 85%, 90%, 95%, 99% or 100% identity to the full length amino acid sequence set forth in SEQ ID N°6 (herein after Xyn_10).
Glycoside hydrolase family 10 ("GH10") enzymes have an (α / β)₈-barrel structure. They hydrolyze internal glycosidic bonds found in carbohydrate polymers and are thus known as endo-acting enzymes that operate via a retaining mechanism, which implies a classical Koshland double-displacement mechanism and that uses at least one acidic catalytic residue Glu as nucleophile and another one generally acting as acid/base catalyst (Pell et al., J. Biol. Chem., 2004, 279(10): 9597-9605). GH10 enzymes may be found in every kingdom, including eukaryote, prokaryote, microbial, fungal and algae. In particular embodiments, the xylanase of GH10 is extracted from the Microbial community of insect's digestive tract.

In particular embodiments, the composition further comprises additional enzyme(s), preferably one or more or all of the following enzymes:
- a β-xylosidase;
- a glucanase;
- an arabinanase; and/or
- an esterase.

In a particular embodiment, the composition comprises a β-xylosidase, preferably a β-xylosidase of Glycoside Hydrolase family 43 (GH43). Advantageously, the β-xylosidase has at least 55%, 60%, 65%, 70%, 75%, 80%, 85%, 90%, 95%, 99% or 100% identity to the full length amino acid sequence set forth in SEQ ID N°8 (*Bh*Xyl)*.*
Glycoside hydrolase family 43 ("GH43") family enzymes display a five-bladed-β-propeller-like structure. The propeller-like structure is based upon a five-fold repeat of blades composed of four-stranded β-sheets. The GH43 enzymes act by inverting mechanism which implies that at least three catalytic amino acids are required. GH43 enzymes may be found in every kingdom, including eukaryote, prokaryote, microbial, fungal and algae. In particular embodiments, the β-xylosidase of GH43 is extracted from the Microbial community of insect's digestive tract.

Alternatively or in addition, the composition comprises a GH3 β-xylosidase. Advantageously, the β-xylosidase has at least 55%, 60%, 65%, 70%, 75%, 80%, 85%, 90%, 95%, 99% or 100% identity to the full length amino acid sequence set forth in SEQ ID N°10 (Xyl_3).

In a particular embodiment, the composition comprises at least one glucanase, preferably a glucanase of Glycoside Hydrolase family 8 (GH8). Advantageously, the glucanase has at least 55%, 60%, 65%, 70%, 75%, 80%, 85%, 90%, 95%, 99% or 100% identity to the full length amino acid sequence set forth in SEQ ID N°14 (Gln_8). Alternatively or in addition, the composition comprises a glucanase having at least 55%, 60%, 65%, 70%, 75%, 80%, 85%, 90%, 95%, 99% or 100% identity to the full length amino acid sequence set forth in SEQ ID N° 20 (Gln_8-20).

In a further embodiment, the composition comprises an arabinanase, preferably an arabinanase of Glycoside Hydrolase family 43 (GH43). Advantageously, the arabinanase has at least 55%, 60%, 65%, 70%, 75%, 80%, 85%, 90%, 95%, 99% or 100% identity to the full length amino acid sequence set forth in SEQ ID N°12 (Abn_43).

In a further particular embodiment, the composition comprises an esterase, preferably an esterase of Carbohydrate Esterase family 1 (CE1).
CE's are a group of enzymes that catalyze the de-O or de-N-acylation of substituted saccharides. The CAZy database has grouped CEs into 16 families, based on sequence similarity. CE1 enzymes may be found in every kingdom, including eukaryote, prokaryote, microbial, fungal and algae. Advantageously, the esterase in the composition has at least 55%, 60%, 65%, 70%, 75%, 80%, 85%, 90%, 95%, 99% or 100% identity to the full length amino acid sequence set forth in SEQ ID N°16 (CE_1-16). Alternatively or in addition, the composition comprises an esterase having at least 55%, 60%, 65%, 70%, 75%, 80%, 85%, 90%, 95%, 99% or 100% identity to the full length amino acid sequence set forth in SEQ ID N°18 (CE_1-18).

In a particular embodiment, the composition comprises a polypeptide Pax, a GH11 xylanase, preferably Xyn_11, and a GH43 β-xylosidase, preferably *Bh*Xyl*.*
In another embodiment, the composition comprises a polypeptide Pax, a GH10 xylanase, preferably Xyn_10, and a GH43 β-xylosidase, preferably *Bh*Xyl*.*

In a particular embodiment, the composition comprises a polypeptide Pax, a GH10 xylanase, preferably Xyn_10, and a GH3 β-xylosidase, preferably (Xyl_3).
In another embodiment, the composition comprises a polypeptide Pax, a GH11 xylanase, preferably Xyn_11, and a GH3 β-xylosidase, preferably (Xyl_3).

In a particular embodiment, the composition comprises a polypeptide Pax, a GH10 xylanase, preferably Xyn_10, and/or a GH11 xylanase, preferably Xyn_11, and a GH8 glucanase, preferably Gln_8 or Gln_8-20.

In a further embodiment, the composition comprises a polypeptide Pax, a GH10 xylanase, preferably Xyn_10, and/or a GH11 xylanase, preferably Xyn_11, a GH43 β-xylosidase, preferably *Bh*Xyl*,* a GH8 glucanase, preferably Gln_8 or Gln_8-20, and a GH43 arabinanase, preferably Abn_43.

In a further embodiment, the composition comprises a polypeptide Pax, a GH10 xylanase, preferably Xyn_10, and/or a GH11 xylanase, preferably Xyn_11, a GH43 β-xylosidase, preferably *Bh*Xyl, a GH8 glucanase, preferably Gln_8 or Gln_8-20, a GH43 arabinanase, preferably Abn_43, and a CE1 esterase, preferably CE_1-18 or CE_1-16.

In a further embodiment, the composition of the invention further comprises a classical commercial enzyme such as a cellulase or a commercial hemicellulase cocktail.

The compositions of the invention comprise various relative ratios of the above described enzymes. In particular, the compositions may comprise between 2 % by weight and 98 % by weight of each enzyme, preferably between 2% and 80% by weight. For instance, the composition comprises about 67 % by weight of the polypeptide Pax and 33 % by weight of a GH10 xylanase, preferably Xyn_10, or GH11 xylanase, preferably Xyn_11.

In another embodiment, the composition comprises a same amount of each of the polypeptides. For instance, the composition comprises about 33 % by weight of the polypeptide Pax, 33 % by weight of a GH10 xylanase, preferably Xyn_10, or GH11, preferably Xyn_11, and 33 % by weight of a GH43 β-xylosidase, preferably *Bh*Xyl*.*
In a further embodiment, the composition comprises about 14 % by weight of the polypeptide Pax, 14 % by weight of a GH43 β-xylosidase, preferably *Bh*Xyl*,* 14 % by weight of a GH10 xylanase, preferably Xyn_10, 14 % by weight of a GH11 xylanase, preferably Xyn_11, 14 % by weight of a GH8 glucanase, preferably Gln_8 or Gln_8-20, 14 % by weight of a CE1 esterase , preferably CE_1-18 or CE_1-16 and 14 % by weight of an GH43 arabinanase, preferably Abn_43.
The amounts and nature of the polypeptides will be easily adapted by those skilled in the art depending e.g., on the nature of the enzymes, the biomass and the fermentable sugars to produce.

The compositions of the invention may further comprise additional polypeptide(s) exhibiting enzymatic activity, and more particularly a hemicellulosic activity. For instance, the compositions may comprise an additional arabinofuranosidase, such as arabinofuranosidase of GH51 (SEQ ID N°22 - Abf_51).

It is a further object of the invention to provide an enzymatic composition comprising a GH51 arabinofuranosidase. In this regards, the invention further relates to a composition comprising such GH51 arabinofuranosidase and at least a xylanase, and uses thereof for modifying a hemicellulose-containing biomass. More particularly, it is an object of the invention to provide an enzymatic mixture comprising the GH51 arabinofuranosidase, a GH10 xylanase, preferably Xyn_10, or a GH11 xylanase, preferably Xyn_11. In a particular embodiment, the composition comprises the GH51 arabinofuranosidase, a GH10 xylanase, preferably Xyn_10, a GH11 xylanase, preferably Xyn_11, a GH43 β-xylosidase, preferably *Bh*Xyl*,* a GH8 glucanase, preferably Gln_8 or Gln_8-20, a CE1 esterase, preferably CE_1-18 or CE_1-16 and a GH43 arabinanase, preferably Abn_43.

Polypeptides and enzymes for use in the invention can be obtained using known techniques for a variety of available sources. For instance, they may be isolated from naturally-occurring sources. For example, one or more polypeptides can be purified or substantially purified from naturally-occurring sources such as microbial environment of animal, human, and insect digestive tract, form soil, sea, fungi, vertebrates, etc. Alternatively, the polypeptides can be recombinantly produced by a host cell, such as by a recombinant bacterium or fungus. The polypeptides may also be prepared by chemical synthesis using conventional methods known per se in the art.

The compositions of the invention may comprise the polypeptides in isolated and/or purified form, and/or a host cell expressing at least one of the polypeptides, and/or an extract thereof. An "*extract of a host cell*" designates any fraction obtained from a host cell, such as a cell supernatant, cell debris, cell walls, DNA extract, enzymes or enzyme preparation or any preparation derived from host cells by chemical, physical and/or enzymatic treatment, which is essentially free of living cells. Preferred extracts are enzymatically-active extracts.

The composition or mixture of the invention may comprise one or several host cells of the invention or extract thereof, and optionally one or several additional cells. In particular, additional cells may be cellulolytic microorganisms (e.g. cellulase, xylanase producers) or fermenting microorganisms (e.g. lactic acid producers, etc.).

In a particular embodiment, the compositions of the invention comprise one or several natural or synthetic polypeptides with hemicellulolytic activities and one or several host cells that produce recombinant polypeptides with hemicellulolytic activities, or extract thereof.

### Nucleic acids

The present invention also relates to nucleic acids encoding the enzymes as disclosed above. The nucleic acids can be DNA (cDNA or gDNA), RNA, or a mixture of the two. It can be in single stranded form or in duplex form or a mixture of the two. It can comprise modified nucleotides, comprising for example a modified bond, a modified purine or pyrimidine base, or a modified sugar. It can be prepared by any method known to one skilled in the art, including chemical synthesis, recombination, and mutagenesis.

More particularly, the present invention relates to
- SEQ ID N°1 that encodes the polypeptide Pax,
- SEQ ID N°3 that encodes the xylanase Xyn_11,
- SEQ ID N°5 that encodes the xylanase Xyn_10,
- SEQ ID N°7 that encodes the β-xylosidase *Bh*Xyl*,*
- SEQ ID N°9 that encodes the β-xylosidase Xyl_3,
- SEQ ID N°11 that encodes the arabinanase Abn_43,
- SEQ ID N°13 and SEQ ID N° 19 that encode the glucanases Gln_8 and Gln_8-20 respectively, and
- SEQ ID N°15 and SEQ ID N°17 that encode the esterases CE_1-16 and CE_1-18 respectively.

Alternatively, the nucleic acids according to the invention may be deduced from the sequences of the polypeptides according to the invention and codon usage may be adapted according to the host cell in which the nucleic acids shall be transcribed. These steps may be carried out according to methods well known to one skilled in the art and some of which are described in the reference manual Sambrook *et al.* (Sambrook *et al.,* 2001).

The present invention further relates to an expression cassette comprising a nucleic acid as described, operably linked to one or more control sequences that direct the expression of said nucleic acid in a suitable host cell. Typically, the expression cassette comprises, or consists of, a nucleic acid according to the invention operably linked to a transcriptional promoter and a transcription terminator.

The present invention also relates to an expression vector comprising such a nucleic acid or such an expression cassette. Said expression vector may be used to transform a host cell and enable the expression of the nucleic acid of the invention in said cell. The choice of the vector will typically depend on the compatibility of the vector with the host cell into which the vector is to be introduced. The vectors may be constructed by the classical techniques of molecular biology, well known to one skilled in the art.

The present invention further relates to the use of such a nucleic acid, an expression cassette or an expression vector to transform, transfect or transduce a cell. The present invention also relates to a host cell comprising such a nucleic acid, a cassette or an expression vector. The host cell may be transformed, transfected or transduced in a transient or stable manner. The expression cassette or vector is introduced into a host cell so that the cassette or vector is maintained as a chromosomal integrant or as a self-replicating extra-chromosomal vector. The term "*host cell*" also encompasses any progeny of a parent host cell that is not identical to the parent host cell due to mutations that occur during replication.

In a particular embodiment, the composition comprises at least one host cell expressing at least one of said polypeptides having an enzymatic activity, or an extract thereof.

In some aspects, the present invention provides a host cell engineered to express one or more or all of the nucleic acids among SEQ ID N°1, SEQ ID N°3, SEQ ID N°5, SEQ ID N°7, SEQ ID N°9, SEQ ID N°11, SEQ ID N°13, SEQ ID N°15, SEQ ID N°17, and SEQ ID N°19, or expression cassettes thereof.

The host cell is desirably a bacterial cell, a mammalian cell, a fungal cell, a yeast cell, an insect cell or a plant cell. In a particular embodiment, the host cell is a fermenting microorganism, preferably selected from the group consisting of bacteria, yeasts and fungi. For instance, the host cell is selected from the group consisting of lactic bacteria. In another embodiment, the host cell is *E. coli.*

In a particular embodiment of the invention, the host cell comprises a nucleotide sequence encoding a polypeptide Pax having at least 55%, 60%, 65%, 70%, 75%, 80%, 85%, 90%, 95%, 99% or 100% identity to the full length amino acid sequence set forth in SEQ ID N°2.

In another particular embodiments, the host cell may further comprise
- a nucleotide sequence encoding a xylanase, preferably a GH10 or GH11 xylanase, having preferably at least 70%, 55%, 60%, 65%, 75%, 80%, 85%, 90%, 95%, 99% or 100% identity to the full length amino acid sequence set forth in SEQ ID N°6 or SEQ ID N°4, and/or
- a nucleotide sequence encoding a β-xylosidase, preferably a GH43 or GH3 β-xylosidase, having preferably at least 55%, 60%, 65%, 70%, 75%, 80%, 85%, 90%, 95%, 99% or 100% identity to the full length amino acid sequence set forth in SEQ ID N°8 or SEQ ID N°10, and/or
- a nucleotide sequence encoding an arabinanase, preferably a GH43 arabinanase, having preferably at least 55%, 60%, 65%, 70%, 75%, 80%, 85%, 90%, 95%, 99% or 100% identity to the full length amino acid sequence set forth in SEQ ID N°12, and/or
- a nucleotide sequence encoding an esterase, preferably a CE1esterase, having preferably at least 55%, 60%, 65%, 70%, 75%, 80%, 85%, 90%, 95%, 99% or 100% identity to the full length amino acid sequence set forth in SEQ ID N°16 or SEQ ID N°18, and/or
- a nucleotide sequence encoding a glucanase, preferably a GH8 glucanase, having preferably at least 55%, 60%, 65%, 70%, 75%, 80%, 85%, 90%, 95%, 99% or 100% identity to the full length amino acid sequence set forth in SEQ ID N°14 or SEQ ID N°20.

In a particular embodiment, the host cell is a recombinant fermenting microorganism that expresses the polypeptide Pax and one or more polypeptides selected from the group consisting of a GH10 xylanase, a GH11 xylanase, a GH43 β-xylosidase, a GH3 β-xylosidase, a GH43 arabinanase, a CE1 esterase and a GH8 glucanase . Advantageously the recombinant fermenting microorganism is a lactic acid bacterium capable of producing lactic acid from the fermentable sugars.

### Hemicellulose containing biomass degradation

The nucleic acids, cassettes, vectors, host cells, compositions and mixtures of the present invention may find use in a variety of industrial applications, including in the degradation of biomass into fermentable sugars.
The present invention thus relates to the use of such nucleic acids, cassettes, vectors, host cells, compositions and mixtures for hydrolysis of a biomass containing hemicellulose. The invention further relates to the use of nucleic acids, cassettes, vectors, host cells, compositions and mixtures of the invention for converting the hemicellulosic fraction of biomass to fermentable sugars, wherein the biomass is contacted with host cells, compositions or mixtures of the invention.
More particularly, the present invention relates to the use of the polypeptide Pax, or compositions comprising such polypeptide Pax, for the hydrolysis of a hemicellulose containing biomass.

In a particular embodiment, the polypeptide Pax may be used with one or more or all of the additional polypeptides selected from
- a xylanase, preferably a GH11 or GH10 xylanase, having preferably at least 55%, 60%, 65%, 70%, 75%, 80%, 85%, 90%, 95%, 99% or 100% identity to the full length amino acid sequence set forth in SEQ ID N°4 or SEQ ID N°6 respectively;
- a β-xylosidase, preferably a GH43 or GH3 β-xylosidase, having preferably at least 55%, 60%, 65%, 70%, 75%, 80%, 85%, 90%, 95%, 99% or 100% identity to the full length amino acid sequence set forth in SEQ ID N°8 or SEQ ID N°10 respectively;
- a glucanase, preferably a GH8 glucanase, having preferably at least 55%, 60%, 65%, 70%, 75%, 80%, 85%, 90%, 95%, 99% or 100% identity to the full length amino acid sequence set forth in SEQ ID N°14 or SEQ ID N°20,
- an arabinanase, preferably a GH43 arabinanase, having preferably at least 55%, 60%, 65%, 70%, 75%, 80%, 85%, 90%, 95%, 99% or 100% identity to the full length amino acid sequence set forth in SEQ ID N°12, and
- an esterase, preferably a CE1 esterase, having preferably at least 55%, 60%, 65%, 70%, 75%, 80%, 85%, 90%, 95%, 99% or 100% identity to the full length amino acid sequence set forth in SEQ ID N°16 or 18.

According to the invention, the polypeptides may be used simultaneously, i.e. all the polypeptides are contacted at the same time, or almost the same time, with the biomass. Alternatively, the polypeptides may be used sequentially, i.e. the polypeptides are sequentially added to the biomass, or in two or more time.

It is another object of the invention to provide a method for modifying a hemicellulose containing biomass, wherein said biomass is contacted with a polypeptide having at least 55%, 60%, 65%, 70%, 75%, 80%, 85%, 90%, 95%, 99% or 100% identity to the full length amino acid sequence set forth in SEQ ID N°2.

Preferably, the biomass is further contacted with a xylanase.

In a particular embodiment, the biomass is contacted with one or more or all of the additional polypeptides selected from
- a xylanase, preferably a GH11 or GH10 xylanase, having preferably at least 55%, 60%, 65%, 70%, 75%, 80%, 85%, 90%, 95%, 99% or 100% identity to the full length amino acid sequence set forth in SEQ ID N°4 or 6 respectively;
- a β-xylosidase, preferably GH43 or GH3 β-xylosidase, having preferably at least 55%, 60%, 65%, 70%, 75%, 80%, 85%, 90%, 95%, 99% or 100% identity to the full length amino acid sequence set forth in SEQ ID N°8 or 10 respectively;
- a glucanase, preferably a GH8 glucanase, having preferably at least 55%, 60%, 65%, 70%, 75%, 80%, 85%, 90%, 95%, 99% or 100% identity to the full length amino acid sequence set forth in SEQ ID N°14 or SEQ ID N°20,
- an arabinanase, preferably a GH43 arabinanase, having preferably at least 55%, 60%, 65%, 70%, 75%, 80%, 85%, 90%, 95%, 99% or 100% identity to the full length amino acid sequence set forth in SEQ ID N°12, and
- an esterase, preferably a CE1 esterase, having preferably at least 55%, 60%, 65%, 70%, 75%, 80%, 85%, 90%, 95%, 99% or 100% identity to the full length amino acid sequence set forth in SEQ ID N°16 or 18.

The polypeptide Pax and/or the compositions of the invention may be used with any kind of biomass that comprises an effective amount of hemicellulose. Advantageously, the biomass comprises at least 10% by weight, preferably 20% by weight, of hemicellulose. In a particular embodiment, the biomass comprises at least 30% by weight of xylane and/or heteroxylane.

In a particular embodiment, the biomass is pretreated before to be contacted with the enzyme(s), host cell, mixture or composition. The pretreatment is intended to disorganize the crystalline structure to access the polymer chains of cellulose and hemicellulose and/or modify the pores in the material to allow the enzymes to penetrate into the fibers to render them amenable to enzymatic hydrolysis. Pretreatments may be mechanical, physical, chemical or biological treatments and are well-known by the skilled person. For example, vegetal biomass may be subjected to pretreatment including ammonia fiber expansion (AFEX), steam explosion, treatment with alkaline aqueous solutions, acidic solutions, organic solvents, ionic liquids (IL), electrolyzed water, phosphoric acid, microwave radiations and combinations thereof. Pretreatments that remove lignin from the biomass may increase the overall amount of sugar released from the hemicellulose.

In another particular embodiment, the mixture or composition comprises about 0.5 g to about 50 g, preferably about 0.5 g to about 20 g, of each of the polypeptides having an enzymatic activity per kilogram of biomass.

Advantageously, at least 10%, 20%, 30%, 40%, 50%, 60%, 70%, 75%, 80%, 85%, 90%, 95% or 100% of the sugars contained in the biomass are degraded.

The methods of the present invention can be practiced at any pH and temperature at which hemicellulose can be degraded. For instance, the methods of the invention are practiced in a pH range of about 5 to about 6 and in a temperature range of about 25 to about 40° C.

Preferably, the methods of the invention are performed in a reactor. "*Reactor*" designates any device or installation or facility suitable for maintaining and transforming biomass. A reactor may comprise inlet and outlet devices to supply/collect medium, nutrients, gas, etc. The reactor may be closed or open, such as a tank.

The present invention also relates to a kit for hydrolyzing hemicellulose, comprising at least
- a polypeptide having at least 55%, 60%, 65%, 70%, 75%, 80%, 85%, 90%, 95%, 99% or 100% identity to the full length amino acid sequence set forth in SEQ ID N°2; and
- a xylanase, preferably a GH11 or GH10 xylanase, with preferably at least 55%, 60%, 65%, 70%, 75%, 80%, 85%, 90%, 95%, 99% or 100% identity to the full length amino acid sequence set forth in SEQ ID N°4 or SEQ ID N°6 respectively.

In a particular embodiment, the kit may further comprise a β-xylosidase, preferably a GH43 or GH3β-xylosidase , having preferably at least 55%, 60%, 65%, 70%, 75%, 80%, 85%, 90%, 95%, 99% or 100% identity to the full length amino acid sequence set forth in SEQ ID N°8 or SEQ ID N°10 respectively.

In another embodiment, the kit may further comprise one or more or all of the additional polypeptides selected from
- a glucanase, preferably a GH8 glucanase, having preferably at least 55%, 60%, 65%, 70%, 75%, 80%, 85%, 90%, 95%, 99% or 100% identity to the full length amino acid sequence set forth in SEQ ID N°14 or SEQ ID N°20,
- an arabinanase, preferably a GH43 arabinanase, having preferably at least 55%, 60%, 65%, 70%, 75%, 80%, 85%, 90%, 95%, 99% or 100% identity to the full length amino acid sequence set forth in SEQ ID N°12, and
- an esterase, preferably a CE1 esterase, having preferably at least 55%, 60%, 65%, 70%, 75%, 80%, 85%, 90%, 95%, 99% or 100% identity to the full length amino acid sequence set forth in SEQ ID N°16 or SEQ ID N°18.

The polypeptides of the kit may be used simultaneously or sequentially.

### Fermentable sugars

It is a further object of the invention to provide a method of producing fermentable sugars from a hemicellulose containing biomass, comprising contacting the biomass with a host cell, mixture, composition or polypeptide(s) of the invention.

It is therefore another object of the invention to provide a method for modifying a hemicellulose containing biomass, comprising the step of:
a) contacting such biomass with a host cell, polypeptide(s) or composition of the invention ;
b) optionally recovering the fermentable sugars from the modified biomass of step a), and
c) optionally fermenting the fermentable sugars of step a) or b).

The recovery of the fermentable sugars includes, e.g., recovery of the fermentation broth comprising the fermentable sugars. The fermentation broth may be used with minimum post-production processing, e.g., purification, ultrafiltration, a cell kill step, etc., and in that case it is said that the fermentation broth is used in a whole broth formulation. Alternatively, the fermentable sugars can be recovered using further purification step(s).

In a particular embodiment, step c) of the method is implemented by contacting the fermentable sugars or biomass containing such fermentable sugars with a fermenting microorganism and producing a fermentation product.

Examples of fermentation products include, but are not limited to, biofuel ; organic acids such as lactic acid, succinic acid, citric acid, adipic acid, itaconic acid, levulinic acid, gluconic acid, acrylic acid, acetic acid, fumaric acid, malic acid, sebacic acid, terephthalic acid, furan-2,5-dicarboxylic acid, propanoïc acid, hydroxypropanoïc acid, hydroxyacids, ethanoïc acid, glycolic acid, glutaric acid, glucaric acid or butanoïc acid; diols such as butanediol, 1,2-propylene glycol, propane-1,3-diol, or monoethylene glycol; amino acids such as aspartic acid, glutamine, glutamic acid, methionine, threonine, lysine, tryptophan or arginine; gaseous olefins such as ethylene, propylene, n-butenes, butadiene, isobutylene or isoprene; vitamins; antibiotics; isosorbide, sorbitol, xylitol, glycerol; polyhydroxyalcanoates; acetone or hydroxymethylfurfural.
Biofuel may be selected from the group consisting of ethanol, butanol, iso-butanol, propanol, isopropanol, methanol, 2,5-dimethylfuran, gamma-valerolactone. Preferably, biofuel is ethanol. Ethanol may be used as a fuel additive or extender in blends of from less than 1% and up to 100% (a fuel substitute). The ethanol-producing fermenting microorganism may be any microorganism known in the art that produces ethanol as fermentation product.

Lactic acid is largely used in the food and packaging industries. Lactic acid may be obtained by fermentation, for example, using bacteria belonging to the genus *Lactococcus, Lactobacillus, Streptococcus* or *Leuconostoc.*

In a particular embodiment, step a) and step c) are performed sequentially.
Alternatively, step a) and step c) may be performed simultaneously. For instance, the mixture or composition comprises at least one host cell chosen to be able to ferment fermentable sugars and that is transformed with a nucleic acid, expression cassette or expression vector as described above, to obtain a modified fermenting strain exhibiting hemicellulolytic properties. This type of strains allows to use a single microorganism community for both hemicellulose hydrolysis and fermentation. In a particular embodiment, the fermenting microorganism is a lactic acid bacterium capable of producing lactic acid from the hydrolysate. Alternatively, the fermentation may be carried out by a second microorganism, introduced in the reactor together with the biomass and/or with the mixture/composition of the invention.

Further aspects and advantages of the invention will be disclosed in the following examples, which should be considered as illustrative and do not limit the scope of this application.

### EXAMPLES

### Example 1: Enzyme Gene Cloning from metagenomic DNA

Standard molecular genetic techniques are used for the cloning and transformation in strain *E. coli* (Sambrook et al. 1989).

Genes comprising sequences SEQ ID N°1, SEQ ID N°5, SEQ ID N°9, SEQ ID N°11, SEQ ID N°13, SEQ ID N°15, SEQ ID N°17, SEQ ID N°19 and SEQ ID N°21 (nucleic acid sequence encoding an arabinofuranosidase of GH51, herein after "Abf_51"), from metagenomic DNA "termite *gut Pseudacantotermes militaris* metagenomic library" were amplified by PCR using the primers of SEQ ID N°23 to SEQ ID N°40.

PCR products were cloned via the gateway system into plasmid pDEST17 with HisTAG or pETG20A containing His and His-Thioredoxin TAG (life-technology) following protocol published by Vincentelli *et al* 2003. Amplified vector was purified using QIAprep Spin Miniprep Kit (Qiagen) and checked by sequencing. The following abbreviations are interchangeably used in the present disclosure:
- vector pDEST17-GH10, producing the xylanase of GH10 (Xyn_10) as set forth in SEQ ID N°6;
- vector pETG20A-GH3, producing the xylosidase of GH3 (Xyl_3) as set forth in SEQ ID N°10;
- vector pETG20A-GH3, producing the polypeptide Abf_3 as set forth in SEQ ID N°2;
- vector pETG20A-GH51, producing the arabinofuranosidase of GH51 (Abf_51) as set forth in SEQ ID N°22;
- vector pETG20A-GH43, producing the arabinanase of GH43 (Abn_43) as set forth in SEQ ID N°12;
- vector pETG20A-GH8-14, producing the glucanase of GH8 (Gln_8-14, or Gln_8) as set forth in SEQ ID N°14;
- vector pETG20A-GH8-20, producing the glucanase of GH8 (Gln_8-20) as set forth in SEQ ID N°20;
- vector pETG20A-CE1-16, producing the esterase of CE1 (CE_1-16) as set forth in SEQ ID N°16;
- vector pETG20A-CE1-18, producing the esterase of CE1 (CE_1-18) as set forth in SEQ ID N°18.

Vector pDEST17 and pETG20A-X were transformed in *E. coli* Rosetta DE3 pLysS (EMD, millipore) and strains expressing the genes were selected on LB Agar + ampicillin. After transformation, transformed bacteria were spread on LB Agar medium containing ampicillin and chloramphenicol (100 and 12.5 µg/mL respectively). Positive colonies were cultivated one night at 37°C in LB medium containing ampicillin and chloramphenicol (100 and 12.5 µg/mL respectively) and used for expression culture.

The vector producing the GH11 xylanase (*Np*Xyn or Xyn_11) as set forth in SEQ ID N°4 was obtained according to the technology disclosed in Vardakou et al. 2008 ("Understanding the Structural Basis for Substrate and Inhibitor Recognition in Eukaryotic GH11 Xylanases" Journal of Molecular Biology 375:1293.)
The vector producing the GH43 xylosidase (*Bh*Xyl or Xyl_43) as set forth in SEQ ID N°8 was obtained according to the technology disclosed in Smaali et al. 2006 ("Expression in Escherichia coli and characterization of beta-xylosidases GH39 and GH-43 from Bacillus halodurans C-125" Appl Microbiol Biotechnol 73:582-90.).

Strains were grown on 2 L flask using a 1.0 L working volume of ZYP5052 auto-inducible medium containing (composition below). Temperature was maintained at 37 °C for 4h and then at 17°C for 16h.

**Table 1: Composition of medium ZYP5052**

| | |
|---|---|
| Tryptone | 1 % w/v |
| Yeast extract | 0.5 % w/v |
| Na₂HPO₄ | 50mM |
| KH₂PO₄ | 50mM |
| (NH4)₂SO₄ | 25mM |
| MgSO₄ | 2mM |
| Glycerol | 0.5% w/v |
| Glucose | 0.05% w/v |
| α-lactose | 0.2% w/v |

Recombinant enzymes were produced in *E. coli* and and purified to homogeneity.

### Example 2: Design and activity of compositions comprising 2 enzymes

A first enzymatic composition was developed and tested for the conversion of heteroxylans contained in a biomass.

This composition comprises
- Abf_3; and
- *Np*Xyn (or Xyn_11, a xylanase from family GH11)).

The performance of the composition was studied in enzymatic hydrolysis assays.
As substrate, 20 mg of wheat bran milled at 2 mm were distributed in 2 mL plastic tubes. The total reaction volume was 1 mL; therefore the substrate concentration in the reaction was 2% (w/v).
The enzymatic dosage employed for each enzyme was 3 mg of purified protein/g biomass and the assays were carried out in 50 mM phosphate buffer at pH 6, for 24h, at 30°C and 1100 rpm in a Thermomixer device.
The assays were performed in triplicates and reactions were stopped by heat treatment at 95 °C for 5 min.
Subsequently, the supernatants were recovered after 10 min centrifugation at 14000 rpm and submitted to reducing sugars quantification by dinitrosalicylic acid (DNS) method (Miller, 1959) using a calibration curve of xylose from 0.1 to 1 g/L and measuring the absorbance at 540 mm.
In some cases monomeric sugars concentration was determined by high performance anionic exchange chromatography with pulsed amperometric detection (HPAEC-PAD).
The recovered supernatants were filtered and directly analyzed by HPAEC to quantify monomers production. Also, the supernatants were incubated for 1 h at 100 °C in presence of H₂SO₄ at 4% (w/v) following the NREL protocol.
Acid treatment produces complete hydrolysis of solubilized oligomers and provides an accurate value of total solubilized sugars during the enzymatic reaction.
The sugar concentration was measured using an ICS 3000 dual (Dionex, France), equipped with a Carbo-Pac PA-1 analytical and guard column. 5 µl of sample diluted at the appropriate concentration were injected and the separation was achieved using 500 mM of sodium acetate and 150 mM of NaOH at 1 ml/min flow rate. Calibration was achieved using monosaccharides at a concentration range from 5 to 50 mg/L.

Figure 1 illustrates the effectiveness of the tested composition: as shown, the composition has very potent hydrolytic activity, and the two enzymes cooperate to provide high yield of sugars. In particular, combining *NpXyn* and Abf_3 increased of 34% the reducing sugars obtained using *Np*Xyn alone.
Abf_3 was primarily identified as an arabinofuranosidase enzyme (34.4 IU/mg protein on pNP-arabinofuranose). However, the results of the experimentation further showed that Abf_3 has a significant β-xylosidase activity (19.0 IU/mg protein on pNP-xylopyranose). This dual activity confers a remarkable advantage to this enzyme for the degradation of heteroxylans.

The benefits of the composition of the invention were further illustrated by comparing the above composition to a composition comprising a distinct arabinofuranosidase belonging to family GH51, SEQ ID N° 22, named Abf_51 and a β-xylosidase belonging to family GH43, SEQ ID N°8, named *Bh*Xyl*.*

Figure 1 shows that the composition of the invention comprising *Np*Xyn and Abf_3 produces 9% and 19% more sugars from wheat bran than the combination of *Np*Xyn-Abf_51 and *Np*Xyn-*BhXyl,* respectively.

In order to further demonstrate the advantages of the invention, the composition of the invention Abf_3 and *Np*Xyn (4 and 2mg/g respectively) was compared to a composition of three enzymes comprising xylanase *Np*Xyn (2 mg/g), β-xylosidase *Bh*Xyl (2 mg/g), and arabinofuranosidase Abf_51 (2mg/g). Figure 2 shows that the composition of the invention, although used at 4mg, is at least as potent as the comparative composition comprising 3 enzymes at 6mg, further illustrating the unexpected potency of the invention.

A composition comprising Abf_3 and Xyn_10 (a xylanase from family GH10) would have similar enzymatic activity compared to the above-described composition.

### Example 3: Design and activity of compositions comprising 3 enzymes

Another enzymatic cocktail for the conversion of heteroxylans contained in a biomass containing hemicellulose, was developed, that comprises
- an arabinofuranosidase from family GH3 (Abf_3);
- a xylanase from family GH11 (*Np*Xyn or Xyn_11), and
- a β-xylosidase from family GH43 (*Bh*Xyl*,* or Xyl_43)

The enzymatic hydrolysis assays were carried out at the same conditions described for the cocktail according to Example 2.

Figure 8 illustrates the advantages of combining *Np*Xyn*, Bh*Xyl and Abf_3, which increases in 54% and 73% the reducing sugars release when comparing to the mixture *Np*Xyn*-Bh*Xyl and the *Np*Xyn alone, respectively. In addition, the benefits of the presence in the cocktail of the novel enzyme Abf_3 were demonstrated by comparison with Abf_51.

The cocktail containing *Np*Xyn*, Bh*Xyl and Abf_3 increased sugars production from wheat bran in 19% in comparison with the cocktail constituted by *Np*Xyn*, Bh*Xyl and Abf_51. The sugars yield obtained was 27%.

HPAEC results (Table 2 below) shows the advantages of the combination *Np*Xyn-*Bh*Xyl-Abf_3 regarding glucose, xylose and arabinose production.

**Table 2. HPAEC results for enzymatic hydrolysis assays using different enzyme combinations.**

| | Monomers (mg/L) | | | Total soluble sugars (after acid hydrolysis, mg/L) | | |
|---|---|---|---|---|---|---|
| | Glucose | Xylose | Arabinose | Glucose | Xylose | Arabinose |
| *Np*Xyn | nd | 367 | 11 | 66 | 1901 | 614 |
| *Np*Xyn + *Bh*Xyl | nd | 1156 | 22 | 59 | 1833 | 600 |
| *Np*Xyn + *Bh*Xyl + Abf_3 | 5 | 1592 | 162 | 200 | 2226 | 728 |

| | | | | | | |
|---|---|---|---|---|---|---|
| nd: not detected | | | | | | |

Another enzymatic cocktail for the conversion of heteroxylans contained in a biomass containing hemicellulose, would have similar activity than the one described above, while comprising the following enzymes:
- an arabinofuranosidase from family GH3 (Abf_3);
- a xylanase from family GH10 (Xyn_10) and
- a β-xylosidase from family GH43 (*Bh*Xyl*,* or Xyl_43)

### Example 4: Design and activity of a hemicellulolytic composition comprising 7 enzymes

Complete degradation of complex biomass usually requires a wide array of enzymes. On the basis of the cocktail detailed in Example 2 (*Np*Xyn + Abf_3), a cocktail composed of 7 enzymes was developed to enhance sugars production from non-pretreated wheat bran.
The enzymatic hydrolysis assays were carried out at the same conditions described in Example 2.

A β-xylosidase, a xylanase, a glucanase, an arabinanase and an esterase were sequentially introduced in the composition of Example 2, to obtain a mixture constituted by seven enzymatic activities (figure 3).
The enzymatic dosage employed for each enzyme was 2 mg of purified protein/g biomass; therefore the cocktail contained 14% of each enzymatic component.
The cocktail was defined by seven activities: xylanase *Np*Xyn (1) and arabinofuranosidase Abf_3 (2) from family GH3, β-xylosidase *Bh*Xyl (3) from family GH43, xylanase Xyn_10 (4) from GH10, glucanase Gln8 (5) from family GH8, arabinanase Abn43 (6) from GH43 and esterase (7) CE1-16 from family CE-1.

This composition has very potent and unexpected hydrolytic activity. Indeed, the use of such composition comprising the combination of the seven above described activities yielded 40% of sugars contained in wheat bran.

The enzymatic activities of said composition have been more precisely studied:

### Xylanases

The composition comprises the combination of xylanases from family GH10 and GH11, which have different substrate specificity (Pollet 2010). The supplementation of *Np*Xyn (GH11) with Xyn_10 (50 % of each enzyme in the mixture) increased reducing sugars production by 32%. Table 3, below, shows the results obtained by HPAEC, showing a positive effect of using the combination GH11 (*Np*Xyn)-GH10 (Xyn_10) in xylose and arabinose release.

**Table 3. HPAEC results: effect of xylanase GH10 on the enzymatic hydrolysis of wheat bran**

| | **Monomers (mg/L)** | | | **Total soluble sugars (mg/L)** | | |
|---|---|---|---|---|---|---|
| | **Glucose** | **Xylose** | **Arabinose** | **Glucose** | **Xylose** | **Arabinose** |
| *NP* Xyn | - | 309±125 | 34±4 | 79±26 | 1708±70 | 347±17 |
| *NP* Xyn + Xyn_10 | - | 537±12 | 62±2 | 124±11 | 2077±20 | 395±9 |

### β-xylosidase

The enzyme β-xylosidase *Bh*Xyl from family GH43 was identified as the most appropriate β-xylosidase in the hydrolysis of wheat bran. Notwithstanding, another β-xylosidase from family GH3 named Xyl_3 showed a positive effect in the hydrolysis of wheat bran and it was revealed as an interesting alternative to *Bh*Xyl.

### Glucanase

The main component of wheat bran is arabinoxylan (40%); however there is also a remarkable proportion of glucose (18.6%). Glucose could take part of cellulosic and/or β-glucan polymers which constitute the wheat bran cell walls.

To evaluate the effect of supplementing a glucanase enzyme Gln_8 from family GH8, an enzymatic assay was carried out using wheat bran as substrate and the conditions detailed in example 2.

A dosage of 5 mg purified protein/g biomass of *Np*Xyn was supplemented with 5 mg purified protein/g biomass of Gln_8. *Np*Xyn alone and Gln_8 alone produced 2.26 g/L and 0.47 g/L of reducing sugars, respectively.

The combination of both activities yielded 3.24 g/L of reducing sugars, which confirms the relevant role of this activity in the cocktail and suggests a synergistic effect between both enzymes. HPAEC analysis confirms the great improvement regarding total solubilized glucose (Table 4, below).

Another glucanase from the family GH8, named Gln_8-20 showed a positive effect as well and represent an alternative to the use in the cocktail of Gln_8.

**Table 4. HPAEC results: effect of glucanase activity on the enzymatic hydrolysis of wheat bran.**

| | Monomers (mg/L) | | | Total soluble sugars (mg/L) | | |
|---|---|---|---|---|---|---|
| | Glucose | Xylose | Arabinose | Glucose | Xylose | Arabinose |
| *Np*Xyn | nd | 339 | 14 | 97 | 2305 | 764 |
| *Np*Xyn+Gln_8 | nd | 487 | 174 | 835 | 2407 | 949 |

### Arabinanase

Arabinanase enzymes hydrolyze internal bonds in the arabinan chains. In herbaceous biomass xylan is typically monosubstituted by arabinose residues. Nevertheless, according to the literature and the high proportion of arabinose in the wheat bran, it is not unlikely to find arabinan chains in the wheat bran hemicellulose.

In this context, the effect of adding an arabinanase activity was studied. The enzyme Abn_43 (family GH43), identified as an arabinan-active enzyme was supplemented to the mixture of xylanases *NpXyn* + Xyn_10 using a dosage of 3 mg purified/g biomass for each enzyme. HPAEC results (table 5) show a clear improvement in sugars production by adding Abn_43.

**Table 5. HPAEC results: effect of arabinanase on the enzymatic hydrolysis of wheat bran.**

| | **Monomers (mg/L)** | | | **Total soluble sugars (mg/L)** | | |
|---|---|---|---|---|---|---|
| | **Glucose** | **Xylose** | **Arabinose** | **Glucose** | **Xylose** | **Arabinose** |
| Xylanases | 4 | 537 | 24 | 319 | 2217 | 395 |
| Xylanases + Abn_43 | 5 | 808 | 112 | 617 | 2975 | 592 |

The effect of the arabinanase Abn_43 was also evaluated in combination with the enzymes already selected for the main cocktail (xylanases *NpXyn* and Xyn_10, and glucanase Gln_8), and in combination with an arabinofuranosidase (Abf_3), that could show a synergistic effect with the arabinanase.

A dosage of 3 mg/g substrate for each enzyme was used.

### Esterase

The composition comprises the carbohydrate esterase CE_1-16 (family CE1). Its positive effect was demonstrated in enzymatic hydrolysis assays under the conditions described in example 2. A dosage of 2 mg purified protein/g biomass of CE_1-16 was supplemented to the composition constituted by two xylanases (*Np*Xyn and Xyn_10), glucanase Gln_8, arabinanase Abn_43, β-xylosidase *BhXyl* and arabinofuranosidase Abf_3.

The addition of CE_1-16 improved reducing sugars release by 7%.

Other esterase from family CE1 and named CE_1-18 also showed a positive effect on the enzymatic hydrolysis of wheat bran and represents an alternative to the use in the composition of CE_1-16.

Once defined the composition optimizing sugars production from wheat bran, a supplementary enzymatic hydrolysis assay was carried out to compare the performance of the novel enzyme Abf_3 to classical arabinofuranosidase. In these experiments, started in parallel at the conditions described above, two different arabinofuranosidases, a classical arabinofuranosidase Abf_51, or the arabinofuranosidase Abf_3, were introduced in the enzymatic mixture constituted by *NpXyn, BhXyl,* Xyn_10, Gln_8, Abn_43 and CE1-16 (also called "Cocktail" in Figure 4).

Figure 4 shows the results for these experiments. The presence of Abf_3 in the composition improves the production of reducing sugars by 8.1% comparing to the Abf_51, illustrating the advantages of using this polypeptide instead of other arabinofuranosidases.

### Example 5: Comparisons with a commercial composition

The performance of the composition of the example 4 (*Np*Xyn, Abf_3, *Bh*Xyl, Xyn_10, Gln_8, Abn_43 and CE1-16) was compared with two commercial preparations from Novozymes:
(1) the mixture of Celluclast® (a cellulase) and NS50030 (a xylanase); and
(2) the commercial hemicellulosic cocktail Cellic HTec2®. This product is described as endoxylanase with high specificity toward soluble hemicellulose and a cellulase background, and it is featured to convert hemicellulose to fermentable sugars.
Three experiments, containing the three different compositions, were started in parallel and in triplicates at the same conditions described in example 2.
In order to compare the performance of the three compositions, the same protein loading was employed in all experiments. The method BCA was used to determine the protein content.

Figure 5 shows the results obtained by DNS analysis of supernatants recovered after the 24h reaction. The composition of the example 4 according to an embodiment of the invention (also called "Optimal Cocktail" in Figure 5 and in Figure 6) constituted by seven enzymatic activities improved the reducing sugars production from wheat bran by 19% and 6% comparing to Celluclast-NS50030 and Cellic HTec2®, respectively.

Supernatants from the Cocktail of the invention and Cellic HTec2® reactions were submitted to HPAEC analysis (Table 6). The main advantage of the Cocktail of the invention derived from this invention was the great increase in monomeric xylose and arabinose from wheat bran. This result confirms the advantages to combine the arabinofuranosidase (Abf_3) to an additional β-xylosidase (*Bh*Xyl)*.*

**Table 6. HPAEC results: comparison of the Cocktail of the invention and Cellic HTec2 on the enzymatic hydrolysis of wheat bran.**

| | **Monomers (mg/L)** | | | **Total soluble sugars (mg/L)** | | |
|---|---|---|---|---|---|---|
| | **Glucose** | **Xylose** | **Arabinose** | **Glucose** | **Xylose** | **Arabinose** |
| Cellic HTec2 | 0.51 | 0.80 | 0.17 | 2.36 | 1.05 | 0.66 |
| Cocktail of the invention | 0.02 | 1.94 | 0.43 | 2.54 | 0.90 | 0.68 |

The potential of the Cocktail of the invention to complement a classical cellulase (Celluclast) was also examined.

Three experiments were started in parallel and in triplicates at the same conditions described in example 2. In one of them, only 5 mg protein/biomass of Celluclast® was added. In the other two Celluclast® was supplemented with an equal dosage of Cocktail of the invention and Cellic HTec2®.

As shown in figure 6, supplementation of Celluclast® with a hemicellulase cocktail improved greatly the reducing sugars production. In addition, the Cocktail of the invention improved reducing sugars production by 24% comparing to Cellic HTec2®, obtaining a sugars yield of 47%. HPAEC analysis (Table 7) confirms the advantages of the Cocktail of the invention, which significantly improved the release of monomeric xylose and arabinose from the heteroxylan contained in wheat bran.

**Table 7. HPAEC results: Effect of cellulase supplementation with hemicellulolytic cocktails on the enzymatic hydrolysis of wheat bran.**

| | **Monomers (mg/L)** | | |
|---|---|---|---|
| | **Glucose** | **Xylose** | **Arabinose** |
| Celluclast® | 0.56 | 0.45 | 0.13 |
| Celluclast® + Cellic HTec2® | 0.85 | 1.65 | 0.25 |
| Celluclast® + Cocktail of the invention | 0.80 | 2.98 | 0.56 |

### Example 6: Evaluation of the composition comprising seven activities on a pretreated substrate

In bioconversion processes from lignocellulosic biomass a pretreatment is usually required to increase the accessibility of the enzymes to carbohydrates and optimize sugars yields. The performance of a hemicellulolytic composition derived from example 4 and constituted by *Np*Xyn, Abf_3, *Bh*Xyl, Xyn_10, Gln_8, Abn_43 and CE1-16 was further evaluated on a pretreated substrate.

Wheat bran was submitted to a physico-chemical treatment. 20 mg of wheat bran were incubated for 30 min at 160°C in presence of 0.2% H₂SO₄ (w/w) (1 mL total volume). A pH of 1.9 was determined at the end of the process. The pretreated material was dried at room temperature and subsequently submitted to an enzymatic hydrolysis using the composition of example 4 (2 mg purified protein/g biomass for each enzyme). The enzymatic hydrolysis conditions were the same described in example 2, but using the pretreated wheat bran as substrate. A control enzymatic hydrolysis using the non-pretreated wheat bran as substrate was started in parallel.

The figure 7 shows the results for this experiment. The acid pretreatment produces itself solubilization of sugars contained in wheat bran up to 4.6 g/L. Followed by an enzymatic hydrolysis using the composition of example 4, a total amount of 8,3 g/L reducing sugars was obtained, which corresponds to a sugars yield of 70%.

### REFERENCES

1. Miller GL. 1959. Use of Dinitrosalicylic Acid Reagent for Determination of Reducing Sugar, Analytical Chemistry, 31(3), 426-428;
2. Pollet A, Delcour JA, Courtin CM. 2010. Structural determinants of the substrate specificities of xylanases from different glycoside hydrolase families Critical Reviews in Biotechnology, 1-16, Early Online;
3. Vincentelli R, Cimino A, Geerlof A, Kubo A, Satou Y, Cambillau C. 2011. High-throughput protein expression screening and purification in Escherichia coli. Methods 55:65-72.

## Claims

1. A composition comprising (i) a polypeptide having at least 55%, 60%, 65%, 70%, 75%, 80%, 85%, 90%, 95%, 99% or 100% identity to the full length amino acid sequence set forth in SEQ ID N°2 and exhibiting both an arabinofuranosidase activity and a β-xylosidase activity, and (ii) at least a xylanase.

2. The composition of claim 1, wherein the xylanase is a xylanase of GH11 or GH10, preferably a xylanase having at least 55%, 60%, 65%, 70%, 75%, 80%, 85%, 90%, 95%, 99% or 100% identity to the full length amino acid sequence set forth in SEQ ID N°4 or SEQ ID N°6.

3. The composition of claim 1 or 2, further comprising at least one polypeptide selected from:
- a β-xylosidase, preferably a GH43 or GH3 β-xylosidase, more preferably a β-xylosidase having at least 55%, 60%, 65%, 70%, 75%, 80%, 85%, 90%, 95%, 99% or 100% identity to the full length amino acid sequence set forth in SEQ ID N°8 or SEQ ID N°10;
- a glucanase, preferably a GH8 glucanase , more preferably a glucanase having at least 55%, 60%, 65%, 70%, 75%, 80%, 85%, 90%, 95%, 99% or 100% identity to the full length amino acid sequence set forth in SEQ ID N°14 or SEQ ID N°20;
- an arabinanase, preferably an GH43 arabinanase , more preferably an arabinanase having at least 55%, 60%, 65%, 70%, 75%, 80%, 85%, 90%, 95%, 99% or 100% identity to the full length amino acid sequence set forth in SEQ ID N°12; and/or
- an esterase, preferably a CElesterase , more preferably an esterase having at least 55%, 60%, 65%, 70%, 75%, 80%, 85%, 90%, 95%, 99% or 100% identity to the full length amino acid sequence set forth in SEQ ID N°16 or SEQ ID N°18.

4. The composition of any one of claims 1-3, comprising between 2% and 98% by weight, preferably between 10% and 80% by weight of each of the polypeptides.

5. The composition of any one of claims 1-4, comprising at least one host cell expressing at least one of the polypeptides, or an extract thereof, the host cell being optionally a fermenting microorganism, preferably selected from the group consisting of bacteria, yeasts and fungi.

6. Use of a polypeptide having at least 55%, 60%, 65%, 70%, 75%, 80%, 85%, 90%, 95%, 99% or 100% identity to the full length amino acid sequence set forth in SEQ ID NO: 2 and exhibiting both an arabinofuranosidase activity and a β-xylosidase activity, or of a microorganism expressing said polypeptide, for modifying a hemicellulose-containing biomass.

7. The use of claim 6, for producing fermentable sugars from the hemicellulose-containing biomass, more preferably xylose and arabinose.

8. The use of claim 6 or 7, further comprising at least one polypeptide selected from
- a xylanase, preferably a GH11 or GH10 xylanase, more preferably a xylanase having at least 55%, 60%, 65%, 70%, 75%, 80%, 85%, 90%, 95%, 99% or 100% identity to the full length amino acid sequence set forth in SEQ ID N°4 or SEQ ID N°6;
- a β-xylosidase, preferably a GH43 or GH3 β-xylosidase, more preferably a β-xylosidase having at least 55%, 60%, 65%, 70%, 75%, 80%, 85%, 90%, 95%, 99% or 100% identity to the full length amino acid sequence set forth in SEQ ID N°8 or SEQ ID N°10;
- a glucanase, preferably a GH8 glucanase, more preferably a glucanase having at least 55%, 60%, 65%, 70%, 75%, 80%, 85%, 90%, 95%, 99% or 100% identity to the full length amino acid sequence set forth in SEQ ID N°14 or SEQ ID N°20;
- an arabinanase, preferably a GH43 arabinanase , more preferably an arabinanase having at least 55%, 60%, 65%, 70%, 75%, 80%, 85%, 90%, 95%, 99% or 100% identity to the full length amino acid sequence set forth in SEQ ID N°12; and
- an esterase, preferably a CE1 esterase , more preferably an esterase having at least 55%, 60%, 65%, 70%, 75%, 80%, 85%, 90%, 95%, 99% or 100% identity to the full length amino acid sequence set forth in SEQ ID N°16 or SEQ ID N°18, wherein the polypeptides are used simultaneously or sequentially.

9. A method for modifying a hemicellulose-containing biomass, comprising
a) contacting such a biomass with a polypeptide having at least 55%, 60%, 65%, 70%, 75%, 80%, 85%, 90%, 95%, 99% or 100% identity to the full length amino acid sequence set forth in SEQ ID NO: 2 and exhibiting both an arabinofuranosidase activity and a β-xylosidase activity, or with a microorganism expressing said polypeptide,
b) optionally recovering fermentable sugars from the modified biomass of step a), and
c) optionally fermenting the fermentable sugars of step a) or b).

10. The method of claim 9, wherein step a) and step c) are performed simultaneously or sequentially, and/or wherein the hemicellulose-containing biomass is subjected to a mechanical and/or physical and/or chemical pretreatment.

11. The method of claim 9 or 10, wherein at least one polypeptide selected from
- a xylanase, preferably a GH11 or GH10 xylanase, more preferably a xylanase having at least 55%, 60%, 65%, 70%, 75%, 80%, 85%, 90%, 95%, 99% or 100% identity to the full length amino acid sequence set forth in SEQ ID N°4 or SEQ ID N°6;
- a β-xylosidase, preferably a GH43 or GH3 β-xylosidase, more preferably a β-xylosidase having at least 55%, 60%, 65%, 70%, 75%, 80%, 85%, 90%, 95%, 99% or 100% identity to the full length amino acid sequence set forth in SEQ ID N°8 or SEQ ID N°10;
- a glucanase, preferably a GH8 glucanase, more preferably a glucanase having at least 55%, 60%, 65%, 70%, 75%, 80%, 85%, 90%, 95%, 99% or 100% identity to the full length amino acid sequence set forth in SEQ ID N°14 or SEQ ID N°20;
- an arabinanase, preferably a GH43 arabinanase , more preferably an arabinanase having at least 55%, 60%, 65%, 70%, 75%, 80%, 85%, 90%, 95%, 99% or 100% identity to the full length amino acid sequence set forth in SEQ ID N°12; and
- an esterase, preferably a CE1 esterase , more preferably an esterase having at least 55%, 60%, 65%, 70%, 75%, 80%, 85%, 90%, 95%, 99% or 100% identity to the full length amino acid sequence set forth in SEQ ID N°16 or SEQ ID N°18, are further contacted simultaneously or sequentially with the biomass in step a).

12. The method of any one of claims 9-11, using about 0.5 g to about 50 g, preferably about 0.5 g to about 20 g, of each of the polypeptides per kilogram of biomass.

13. A recombinant lactic acid bacterium comprising a nucleotide sequence encoding a polypeptide having at least 55%, 60%, 65%, 70%, 75%, 80%, 85%, 90%, 95%, 99% or 100% identity to the full length amino acid sequence set forth in SEQ ID N°2 and exhibiting both an arabinofuranosidase activity and a β-xylosidase activity, and optionally a nucleotide sequence encoding a GH10 xylanase having preferably at least 55%, 60%, 65%, 70%, 75%, 80%, 85%, 90%, 95%, 99% or 100% identity to the full length amino acid sequence set forth in SEQ ID N°6, and/or a nucleotide sequence encoding a GH11 xylanase having preferably at least 55%, 60%, 65%, 70%, 75%, 80%, 85%, 90%, 95%, 99% or 100% identity to the full length amino acid sequence set forth in SEQ ID N°4, and/or a nucleotide sequence encoding a GH43 β-xylosidase having preferably at least 55%, 60%, 65%, 70%, 75%, 80%, 85%, 90%, 95%, 99% or 100% identity to the full length amino acid sequence set forth in SEQ ID N°8, and/or a nucleotide sequence encoding a GH3 β-xylosidase having preferably at least 55%, 60%, 65%, 70%, 75%, 80%, 85%, 90%, 95%, 99% or 100% identity to the full length amino acid sequence set forth in SEQ ID N°10, and/or a nucleotide sequence encoding an GH43 arabinanase having preferably at least 55%, 60%, 65%, 70%, 75%, 80%, 85%, 90%, 95%, 99% or 100% identity to the full length amino acid sequence set forth in SEQ ID N°12, and/or a nucleotide sequence encoding an CElesterase having preferably at least 55%, 60%, 65%, 70%, 75%, 80%, 85%, 90%, 95%, 99% or 100% identity to the full length amino acid sequence set forth in SEQ ID N°16 or SEQ ID N°18, and/or a nucleotide sequence encoding a GH8 glucanase having preferably at least 55%, 60%, 65%, 70%, 75%, 80%, 85%, 90%, 95%, 99% or 100% identity to the full length amino acid sequence set forth in SEQ ID N°14 or SEQ ID N°20.

14. A kit for hydrolyzing hemicellulose, comprising at least
- a polypeptide having at least 55%, 60%, 65%70%, 75%, 80%, 85%, 90%, 95%, 99% or 100% identity to the full length amino acid sequence set forth in SEQ ID N°2 and exhibiting both an arabinofuranosidase activity and a β-xylosidase activity; and
- a xylanase, preferably a GH11 or GH10xylanase, with preferably at least 55%, 60%, 65%, 70%, 75%, 80%, 85%, 90%, 95%, 99% or 100% identity to the full length amino acid sequence set forth in SEQ ID N°4 or 6 respectively.

15. The kit of claim 14, further comprising at least one polypeptide selected from
- a xylanase, preferably a GH11 or GH10 xylanase, more preferably a xylanase having at least 55%, 60%, 65%, 70%, 75%, 80%, 85%, 90%, 95%, 99% or 100% identity to the full length amino acid sequence set forth in SEQ ID N°4 or SEQ ID N°6;
- a β-xylosidase, preferably a GH43 or GH3 β-xylosidase, more preferably a β-xylosidase having at least 55%, 60%, 65%, 70%, 75%, 80%, 85%, 90%, 95%, 99% or 100% identity to the full length amino acid sequence set forth in SEQ ID N°8 or SEQ ID N°10;
- a glucanase, preferably a GH8 glucanase, more preferably a glucanase having at least 55%, 60%, 65%, 70%, 75%, 80%, 85%, 90%, 95%, 99% or 100% identity to the full length amino acid sequence set forth in SEQ ID N°14 or SEQ ID N°20;
- an arabinanase, preferably a GH43 arabinanase , more preferably an arabinanase having at least 55%, 60%, 65%, 70%, 75%, 80%, 85%, 90%, 95%, 99% or 100% identity to the full length amino acid sequence set forth in SEQ ID N°12; and
- an esterase, preferably a CE1 esterase , more preferably an esterase having at least 55%, 60%, 65%, 70%, 75%, 80%, 85%, 90%, 95%, 99% or 100% identity to the full length amino acid sequence set forth in SEQ ID N°16 or SEQ ID N°18.

## Patentansprüche

1. Eine Zusammensetzung umfassend (i) ein Polypeptid mit wenigstens 55%, 60%, 65%, 70%, 75%, 80%, 85%, 90%, 95%, 99% oder 100% Identität zu der volllangen Aminosäuresequenz, die in SEQ ID NO: 2 dargestellt ist, und sowohl eine Arabinofuranosidaseaktivität als auch eine β-Xylosidaseaktivität zeigt, und (ii) wenigstens eine Xylanase.

2. Die Zusammensetzung nach Anspruch 1, wobei die Xylanase eine Xylanase von GH11 oder GH10, bevorzugt eine Xylanase mit wenigstens 55%, 60%, 65%, 70%, 75%, 80%, 85%, 90%, 95%, 99% oder 100% Identität zu der volllangen Aminosäuresequenz ist, die in SEQ ID NO: 4 oder SEQ ID NO: 6 dargestellt ist.

3. Die Zusammensetzung nach Anspruch 1 oder 2, außerdem umfassend wenigstens ein Polypeptid, ausgewählt aus:
- einer β-Xylosidase, bevorzugt einer GH43- oder GH3-β-Xylosidase, bevorzugter einer β-Xylosidase mit wenigstens 55%, 60%, 65%, 70%, 75%, 80%, 85%, 90%, 95%, 99% oder 100% Identität zu der volllangen Aminosäuresequenz, die in SEQ ID NO: 8 oder SEQ ID NO: 10 dargestellt ist;
- einer Glucanase, bevorzugt einer GH8-Glucanase, bevorzugter einer Glucanase mit wenigstens 55%, 60%, 65%, 70%, 75%, 80%, 85%, 90%, 95%, 99% oder 100% Identität zu der volllangen Aminosäuresequenz, die in SEQ ID NO: 14 oder SEQ ID NO: 20 dargestellt ist;
- einer Arabinanase, bevorzugt einer GH43-Arabinanase, bevorzugter einer Arabinanase mit wenigstens 55%, 60%, 65%, 70%, 75%, 80%, 85%, 90%, 95%, 99% oder 100% Identität zu der volllangen Aminosäuresequenz, die in SEQ ID NO: 12 dargestellt ist; und/oder
- einer Esterase, bevorzugt einer CE1-Esterase, bevorzugter einer Esterase mit wenigstens 55%, 60%, 65%, 70%, 75%, 80%, 85%, 90%, 95%, 99% oder 100% Identität zu der volllangen Aminosäuresequenz, die in SEQ ID NO: 16 oder SEQ ID NO: 18 dargestellt ist.

4. Die Zusammensetzung nach einem der Ansprüche 1 bis 3, umfassend zwischen 2 Gew.-% und 98 Gew.-%, bevorzugt zwischen 10 Gew.-% und 80 Gew.-% von jedem der Polypeptide.

5. Die Zusammensetzung nach einem der Ansprüche 1 bis 4, umfassend wenigstens eine Wirtszelle, die wenigstens eines der Polypeptide exprimiert, oder einen Extrakt davon, wobei die Wirtszelle gegebenenfalls ein fermentierender Mikroorganismus ist, bevorzugt ausgewählt aus der Gruppe, bestehend aus Bakterien, Hefen und Pilzen.

6. Verwendung eines Polypeptids mit wenigstens 55%, 60%, 65%, 70%, 75%, 80%, 85%, 90%, 95%, 99% oder 100% Identität zu der volllangen Aminosäuresequenz, die in SEQ ID NO: 2 dargestellt ist, und sowohl eine Arabinofuranosidaseaktivität als auch eine β-Xylosidaseaktivität zeigt, oder eines Mikroorganismus, der besagtes Polypeptid exprimiert, zur Modifizierung einer Hemicellulose-haltigen Biomasse.

7. Die Verwendung nach Anspruch 6 zur Herstellung fermentierbarer Zucker, bevorzugter Xylose und Arabinose aus der Hemicellulose-haltigen Biomasse.

8. Die Verwendung nach Anspruch 6 oder 7, außerdem umfassend wenigstens ein Polypeptid, ausgewählt aus
- einer Xylanase, bevorzugt einer GH11- oder GH10-Xylanase, bevorzugter einer Xylanase mit wenigstens 55%, 60%, 65%, 70%, 75%, 80%, 85%, 90%, 95%, 99% oder 100% Identität zu der volllangen Aminosäuresequenz, die in SEQ ID NO: 4 oder SEQ ID NO: 6 dargestellt ist;
- einer β-Xylosidase, bevorzugt einer GH43- oder GH3-β-Xylosidase, bevorzugter einer β-Xylosidase mit wenigstens 55%, 60%, 65%, 70%, 75%, 80%, 85%, 90%, 95%, 99% oder 100% Identität zu der volllangen Aminosäuresequenz, die in SEQ ID NO: 8 oder SEQ ID NO: 10 dargestellt ist;
- einer Glucanase, bevorzugt einer GH8-Glucanase, bevorzugter einer Glucanase mit wenigstens 55%, 60%, 65%, 70%, 75%, 80%, 85%, 90%, 95%, 99% oder 100% Identität zu der volllangen Aminosäuresequenz, die in SEQ ID NO: 14 oder SEQ ID NO: 20 dargestellt ist;
- einer Arabinanase, bevorzugt einer GH43-Arabinanase, bevorzugter einer Arabinanase mit wenigstens 55%, 60%, 65%, 70%, 75%, 80%, 85%, 90%, 95%, 99% oder 100% Identität zu der volllangen Aminosäuresequenz, die in SEQ ID NO: 12 dargestellt ist; und
- einer Esterase, bevorzugt einer CE1-Esterase, bevorzugter einer Esterase mit wenigstens 55%, 60%, 65%, 70%, 75%, 80%, 85%, 90%, 95%, 99% oder 100% Identität zu der volllangen Aminosäuresequenz, die in SEQ ID NO: 16 oder SEQ ID NO: 18 dargestellt ist, wobei die Polypeptide gleichzeitig oder aufeinanderfolgend verwendet werden.

9. Ein Verfahren zur Modifizierung einer Hemicellulose-haltigen Biomasse, umfassend
a) Inkontaktbringen einer derartigen Biomasse mit einem Polypeptid mit wenigstens 55%, 60%, 65%, 70%, 75%, 80%, 85%, 90%, 95%, 99% oder 100% Identität zu der volllangen Aminosäuresequenz, die in SEQ ID NO: 2 dargestellt ist, und sowohl eine Arabinofuranosidaseaktivität als auch eine β-Xylosidaseaktivität zeigt, oder mit einem Mikroorganismus, der besagtes Polypeptid exprimiert,
b) gegebenenfalls Gewinnung fermentierbarer Zucker aus der modifizierten Biomasse von Schritt a), und
c) gegebenenfalls Fermentieren der fermentierbaren Zucker von Schritt a) oder b).

10. Das Verfahren nach Anspruch 9, wobei Schritt a) und Schritt c) gleichzeitig oder aufeinanderfolgend durchgeführt werden und/oder wobei die Hemicellulose-haltige Biomasse einer mechanischen und/oder physikalischen und/oder chemischen Vorbehandlung unterzogen wird.

11. Das Verfahren nach Anspruch 9 oder 10, wobei wenigstens ein Polypeptid, ausgewählt aus
- einer Xylanase, bevorzugt einer GH11- oder GH10-Xylanase, bevorzugter einer Xylanase mit wenigstens 55%, 60%, 65%, 70%, 75%, 80%, 85%, 90%, 95%, 99% oder 100% Identität zu der volllangen Aminosäuresequenz, die in SEQ ID NO: 4 oder SEQ ID NO: 6 dargestellt ist;
- einer β-Xylosidase, bevorzugt einer GH43- oder GH3-β-Xylosidase, bevorzugter einer β-Xylosidase mit wenigstens 55%, 60%, 65%, 70%, 75%, 80%, 85%, 90%, 95%, 99% oder 100% Identität zu der volllangen Aminosäuresequenz, die in SEQ ID NO: 8 oder SEQ ID NO: 10 dargestellt ist;
- einer Glucanase, bevorzugt einer GH8-Glucanase, bevorzugter einer Glucanase mit wenigstens 55%, 60%, 65%, 70%, 75%, 80%, 85%, 90%, 95%, 99% oder 100% Identität zu der volllangen Aminosäuresequenz, die in SEQ ID NO: 14 oder SEQ ID NO: 20 dargestellt ist;
- einer Arabinanase, bevorzugt einer GH43-Arabinanase, bevorzugter einer Arabinanase mit wenigstens 55%, 60%, 65%, 70%, 75%, 80%, 85%, 90%, 95%, 99% oder 100% Identität zu der volllangen Aminosäuresequenz, die in SEQ ID NO: 12 dargestellt ist; und
- einer Esterase, bevorzugt einer CE1-Esterase, bevorzugter einer Esterase mit wenigstens 55%, 60%, 65%, 70%, 75%, 80%, 85%, 90%, 95%, 99% oder 100% Identität zu der volllangen Aminosäuresequenz, die in SEQ ID NO: 16 oder SEQ ID NO: 18 dargestellt ist, außerdem gleichzeitig oder aufeinanderfolgend mit der Biomasse in Schritt a) in Kontakt gebracht werden.

12. Das Verfahren nach einem der Ansprüche 9 bis 11, unter Verwendung von etwa 0,5 g bis etwa 50 g, bevorzugt etwa 0,5 g bis etwa 20 g von jedem der Polypeptide pro Kilogramm Biomasse.

13. Ein rekombinantes Milchsäurebakterium, umfassend eine Nukleotidsequenz, codierend ein Polypeptid mit wenigstens 55%, 60%, 65%, 70%, 75%, 80%, 85%, 90%, 95%, 99% oder 100% Identität zu der volllangen Aminosäuresequenz, die in SEQ ID NO: 2 dargestellt ist, und sowohl eine Arabinofuranosidaseaktivität als auch eine β-Xylosidaseaktivität zeigt, und gegebenenfalls eine Nukleotidsequenz, codierend eine GH10-Xylanase mit bevorzugt wenigstens 55%, 60%, 65%, 70%, 75%, 80%, 85%, 90%, 95%, 99% oder 100% Identität zu der volllangen Aminosäuresequenz, die in SEQ ID NO: 6 dargestellt ist, und/oder eine Nukleotidsequenz, codierend eine GH11-Xylanase mit bevorzugt wenigstens 55%, 60%, 65%, 70%, 75%, 80%, 85%, 90%, 95%, 99% oder 100% Identität zu der volllangen Aminosäuresequenz, die in SEQ ID NO: 4 dargestellt ist, und/oder eine Nukleotidsequenz, codierend eine GH43-β-Xylosidase mit bevorzugt wenigstens 55%, 60%, 65%, 70%, 75%, 80%, 85%, 90%, 95%, 99% oder 100% Identität zu der volllangen Aminosäuresequenz, die in SEQ ID NO: 8 dargestellt ist, und/oder eine Nukleotidsequenz, codierend eine GH3-β-Xylosidase mit bevorzugt wenigstens 55%, 60%, 65%, 70%, 75%, 80%, 85%, 90%, 95%, 99% oder 100% Identität zu der volllangen Aminosäuresequenz, die in SEQ ID NO: 10 dargestellt ist, und/oder eine Nukleotidsequenz, codierend eine GH43-Arabinanase mit bevorzugt wenigstens 55%, 60%, 65%, 70%, 75%, 80%, 85%, 90%, 95%, 99% oder 100% Identität zu der volllangen Aminosäuresequenz, die in SEQ ID NO: 12 dargestellt ist, und/oder eine Nukleotidsequenz, codierend eine CE1-Esterase mit bevorzugt wenigstens 55%, 60%, 65%, 70%, 75%, 80%, 85%, 90%, 95%, 99% oder 100% Identität zu der volllangen Aminosäuresequenz, die in SEQ ID NO: 16 oder SEQ ID NO: 18 dargestellt ist, und/oder eine Nukleotidsequenz, codierend eine GH8-Glucanase mit bevorzugt wenigstens 55%, 60%, 65%, 70%, 75%, 80%, 85%, 90%, 95%, 99% oder 100% Identität zu der volllangen Aminosäuresequenz, die in SEQ ID NO: 14 oder SEQ ID NO: 20 dargestellt ist.

14. Ein Kit zum Hydrolysieren von Hemicellulose, umfassend wenigstens
- ein Polypeptid mit wenigstens 55%, 60%, 65%, 70%, 75%, 80%, 85%, 90%, 95%, 99% oder 100% Identität zu der volllangen Aminosäuresequenz, die in SEQ ID NO: 2 dargestellt ist, und sowohl eine Arabinofuranosidaseaktivität als auch eine β-Xylosidaseaktivität zeigt; und
- eine Xylanase, bevorzugt eine GH11- oder G10-Xylanase mit bevorzugt wenigstens 55%, 60%, 65%, 70%, 75%, 80%, 85%, 90%, 95%, 99% oder 100% Identität zu der volllangen Aminosäuresequenz, die in SEQ ID NO: 4 beziehungsweise 6 dargestellt ist.

15. Das Kit nach Anspruch 14, außerdem umfassend wenigstens ein Polypeptid, ausgewählt aus
- einer Xylanase, bevorzugt einer GH11- oder GH10-Xylanase, bevorzugter einer Xylanase mit wenigstens 55%, 60%, 65%, 70%, 75%, 80%, 85%, 90%, 95%, 99% oder 100% Identität zu der volllangen Aminosäuresequenz, die in SEQ ID NO: 4 oder SEQ ID NO: 6 dargestellt ist;
- einer β-Xylosidase, bevorzugt einer GH43- oder GH3-β-Xylosidase, bevorzugter einer β-Xylosidase mit wenigstens 55%, 60%, 65%, 70%, 75%, 80%, 85%, 90%, 95%, 99% oder 100% Identität zu der volllangen Aminosäuresequenz, die in SEQ ID NO: 8 oder SEQ ID NO: 10 dargestellt ist;
- einer Glucanase, bevorzugt einer GH8-Glucanase, bevorzugter einer Glucanase mit wenigstens 55%, 60%, 65%, 70%, 75%, 80%, 85%, 90%, 95%, 99% oder 100% Identität zu der volllangen Aminosäuresequenz, die in SEQ ID NO: 14 oder SEQ ID NO: 20 dargestellt ist;
- einer Arabinanase, bevorzugt einer GH43-Arabinanase, bevorzugter einer Arabinanase mit wenigstens 55%, 60%, 65%, 70%, 75%, 80%, 85%, 90%, 95%, 99% oder 100% Identität zu der volllangen Aminosäuresequenz, die in SEQ ID NO: 12 dargestellt ist; und
- einer Esterase, bevorzugt einer CE1-Esterase, bevorzugter einer Esterase mit wenigstens 55%, 60%, 65%, 70%, 75%, 80%, 85%, 90%, 95%, 99% oder 100% Identität zu der volllangen Aminosäuresequenz, die in SEQ ID NO: 16 oder SEQ ID NO: 18 dargestellt ist.

## Revendications

1. Composition comprenant (i) un polypeptide ayant au moins 55%, 60%, 65%, 70%, 75%, 80%, 85%, 90%, 95%, 99% ou 100% d'identité avec la séquence complète d'acides aminés présentée dans SEQ ID N°2 et présentant à la fois une activité arabinofuranosidase et une activité β-xylosidase, et (ii) au moins une xylanase.

2. Composition selon la revendication 1, dans laquelle la xylanase est une xylanase de GH11 ou GH10, préférablement une xylanase ayant au moins 55%, 60%, 65%, 70%, 75%, 80%, 85%, 90%, 95%, 99% ou 100% d'identité avec la séquence complète d'acide aminés présentée dans SEQ ID N°4 ou SEQ ID N°6.

3. Composition selon la revendication 1 ou 2, comprenant en outre au moins un polypeptide choisi parmi :
- une β-xylosidase, préférablement une β-xylosidase GH43 ou GH3, plus préférablement une β-xylosidase ayant au moins 55%, 60%, 65%, 70%, 75%, 80%, 85%, 90%, 95%, 99% ou 100% d'identité avec la séquence complète d'acide aminés présentée dans SEQ ID N°8 ou SEQ ID N°10 ;
- une glucanase, préférablement une glucanase GH8, plus préférablement une glucanase ayant au moins 55%, 60%, 65%, 70%, 75%, 80%, 85%, 90%, 95%, 99% ou 100% d'identité avec la séquence complète d'acide aminés présentée dans SEQ ID N°14 ou SEQ ID N°20 ;
- une arabinanase, préférablement une arabinanase GH43, plus préférablement une arabinanase ayant au moins 55%, 60%, 65%, 70%, 75%, 80%, 85%, 90%, 95%, 99% ou 100% d'identité avec la séquence complète d'acide aminés présentée dans SEQ ID N°12 ; et/ou
- une estérase, préférablement une estérase CE1, plus préférablement une estérase ayant au moins 55%, 60%, 65%, 70%, 75%, 80%, 85%, 90%, 95%, 99% ou 100% d'identité avec la séquence complète d'acide aminés présentée dans SEQ ID N°16 ou SEQ ID N°18.

4. Composition selon l'une quelconque des revendications 1 à 3, comprenant entre 2% et 98% en poids, préférablement entre 10% et 80% en poids de chacun des polypeptides.

5. Composition selon l'une quelconque des revendication 1 à 4, comprenant au moins une cellule hôte exprimant au moins un des polypeptides, ou un extrait de celui-ci, la cellule hôte étant éventuellement un microorganisme fermenteur, préférablement choisi dans le groupe constitué par des bactéries, des levures et des champignons.

6. Utilisation d'un polypeptide ayant au moins 55%, 60%, 65%, 70%, 75%, 80%, 85%, 90%, 95%, 99% ou 100% d'identité avec la séquence complète d'acide aminés présentée dans SEQ ID N : 2 et présentant à la fois une activité arabinofuranosidase et une activité β-xylosidase, ou d'un microorganisme exprimant ledit polypeptide, pour modifier une biomasse contenant de l'hémicellulose.

7. Utilisation selon la revendication 6, pour la production de sucres fermentables à partir de la biomasse contenant de l'hémicellulose, plus préférablement le xylose et l'arabinose.

8. Utilisation selon la revendication 6 ou 7, comprenant en outre au moins un polypeptide choisi parmi
- une xylanase, préférablement une xylanase GH11 ou GH10, plus préférablement une xylanase ayant au moins 55%, 60%, 65%, 70%, 75%, 80%, 85%, 90%, 95%, 99% ou 100% d'identité avec la séquence complète d'acide aminés présentée dans SEQ ID N° 4 ou SEQ ID N°6 ;
- une β-xylosidase, préférablement une β-xylosidase GH43 ou GH3, plus préférablement une β-xylosidase ayant au moins 55%, 60%, 65%, 70%, 75%, 80%, 85%, 90%, 95%, 99% ou 100% d'identité avec la séquence complète d'acide aminés présentée dans SEQ ID N°8 ou SEQ ID N°10 ;
- une glucanase, préférablement une glucanase GH8, plus préférablement une glucanase ayant au moins 55%, 60%, 65%, 70%, 75%, 80%, 85%, 90%, 95%, 99% ou 100% d'identité avec la séquence complète d'acide aminés présentée dans SEQ ID N°14 ou SEQ ID N°20 ;
- une arabinanase, préférablement une arabinanase GH43, plus préférablement une arabinanase ayant au moins 55%, 60%, 65%, 70%, 75%, 80%, 85%, 90%, 95%, 99% ou 100% d'identité avec la séquence complète d'acide aminés présentée dans SEQ ID N°12 ; et
- une estérase, préférablement une estérase CE1, plus préférablement une estérase ayant au moins 55%, 60%, 65%, 70%, 75%, 80%, 85%, 90%, 95%, 99% ou 100% d'identité avec la séquence complète d'acide aminés présentée dans SEQ ID N°16 ou SEQ ID N°18, dans laquelle les polypeptides sont utilisés simultanément ou séquentiellement.

9. Méthode pour modifier une biomasse contenant de l'hémicellulose, comprenant
a) la mise en contact d'une telle biomasse avec un polypeptide ayant au moins 55%, 60%, 65%, 70%, 75%, 80%, 85%, 90%, 95%, 99% ou 100% d'identité avec la séquence complète d'acide aminés présentée dans SEQ ID N°2 et présentant à la fois une activité arabinofuranosidase et une activité β-xylosidase, ou avec un microorganisme exprimant ledit polypeptide,
b) éventuellement la récupération de sucres fermentables à partir de la biomasse modifiée de l'étape a), et
c) éventuellement la fermentation des sucres fermentables de l'étape a) ou b).

10. Méthode selon la revendication 9, dans laquelle l'étape a) et l'étape c) sont réalisées simultanément ou séquentiellement, et/ou dans laquelle la biomasse contenant de l'hémicellulose est soumise à un prétraitement mécanique et/ou physique et/ou chimique.

11. Méthode selon la revendication 9 ou 10, dans laquelle au moins un polypeptide choisi parmi
- une xylanase, préférablement une xylanase GH11 ou GH10, plus préférablement une xylanase ayant au moins 55%, 60%, 65%, 70%, 75%, 80%, 85%, 90%, 95%, 99% ou 100% d'identité avec la séquence complète d'acide aminés présentée dans SEQ ID N° 4 ou SEQ ID N°6 ;
- une β-xylosidase, préférablement une β-xylosidase GH43 ou GH3, plus préférablement une β-xylosidase ayant au moins 55%, 60%, 65%, 70%, 75%, 80%, 85%, 90%, 95%, 99% ou 100% d'identité avec la séquence complète d'acide aminés présentée dans SEQ ID N°8 ou SEQ ID N°10 ;
- une glucanase, préférablement une glucanase GH8, plus préférablement une glucanase ayant au moins 55%, 60%, 65%, 70%, 75%, 80%, 85%, 90%, 95%, 99% ou 100% d'identité avec la séquence complète d'acide aminés présentée dans SEQ ID N°14 ou SEQ ID N°20 ;
- une arabinanase, préférablement une arabinanase GH43, plus préférablement une arabinanase ayant au moins 55%, 60%, 65%, 70%, 75%, 80%, 85%, 90%, 95%, 99% ou 100% d'identité avec la séquence complète d'acide aminés présentée dans SEQ ID N°12 ; et
- une estérase, préférablement une estérase CE1, plus préférablement une estérase ayant au moins 55%, 60%, 65%, 70%, 75%, 80%, 85%, 90%, 95%, 99% ou 100% d'identité avec la séquence complète d'acide aminés présentée dans SEQ ID N°16 ou SEQ ID N°18, est en outre en contact simultanément ou séquentiellement avec la biomasse au cours de l'étape a).

12. Méthode selon l'une quelconque des revendication 9 à 11, utilisant environ 0.5 g à environ 50 g, préférablement environ 0.5g à environ 20 g, de chacun des polypeptides par kilogramme de biomasse.

13. Bactérie lactique recombinante comprenant une séquence nucléotidique codant un polypeptide ayant au moins 55%, 60%, 65%, 70%, 75%, 80%, 85%, 90%, 95%, 99% ou 100% d'identité avec la séquence complète d'acide aminés présentée dans SEQ ID N°2 et présentant à la fois une activité arabinofuranosidase et une activité β-xylosidase, et éventuellement une séquence nucléotidique codant une xylanase GH10 ayant préférablement au moins 55%, 60%, 65%, 70%, 75%, 80%, 85%, 90%, 95%, 99% ou 100% d'identité avec la séquence complète d'acide aminés présentée dans SEQ ID N°6, et/ou une séquence nucléotidique codant une xynalase GH11 ayant préférablement au moins 55%, 60%, 65%, 70%, 75%, 80%, 85%, 90%, 95%, 99% ou 100% d'identité avec la séquence complète d'acide aminés présentée dans SEQ ID N°4, et/ou une séquence nucléotidique codant une β-xylosidase GH43 ayant préférablement au moins 55%, 60%, 65%, 70%, 75%, 80%, 85%, 90%, 95%, 99% ou 100% d'identité avec la séquence complète d'acide aminés présentée dans SEQ ID N°8, et/ou une séquence nucléotidique codant une β-xylosidase GH3 ayant préférablement au moins 55%, 60%, 65%, 70%, 75%, 80%, 85%, 90%, 95%, 99% ou 100% d'identité avec la séquence complète d'acide aminés présentée dans SEQ ID N°10, et/ou une séquence nucléotidique codant une arabinanase GH43 ayant préférablement au moins 55%, 60%, 65%, 70%, 75%, 80%, 85%, 90%, 95%, 99% ou 100% d'identité avec la séquence complète d'acide aminés présentée dans SEQ ID N°12, et/ou une séquence nucléotidique codant une estérase CE1 ayant préférablement au moins 55%, 60%, 65%, 70%, 75%, 80%, 85%, 90%, 95%, 99% ou 100% d'identité avec la séquence complète d'acide aminés présentée dans SEQ ID N°16 ou SEQ ID N°18, et/ou une séquence nucléotidique codant une glucanase GH8 ayant préférablement au moins 55%, 60%, 65%, 70%, 75%, 80%, 85%, 90%, 95%, 99% ou 100% d'identité avec la séquence complète d'acide aminés présentée dans SEQ ID N°14 ou SEQ ID N°20.

14. Kit pour hydrolyser l'hémicellulose, comprenant au moins
- un polypeptide ayant au moins 55%, 60%, 65%, 70%, 75%, 80%, 85%, 90%, 95%, 99% ou 100% d'identité avec la séquence complète d'acide aminés présentée dans SEQ ID N°2 et présentant à la fois une activité arabinofuranosidase et une activité β-xylosidase ; et
- Une xylanase, préférablement une xylanase GH11 ou GH10, avec préférablement au moins 55%, 60%, 65%, 70%, 75%, 80%, 85%, 90%, 95%, 99% ou 100% d'identité avec la séquence complète d'acide aminés présentée dans SEQ ID N° 4 ou 6 respectivement.

15. Kit selon la revendication 14, comprenant en outre au moins un polypeptide choisi parmi
- une xylanase, préférablement une xylanase GH11 ou GH10, plus préférablement une xylanase ayant au moins 55%, 60%, 65%, 70%, 75%, 80%, 85%, 90%, 95%, 99% ou 100% d'identité avec la séquence complète d'acide aminés présentée dans SEQ ID N° 4 ou SEQ ID N°6 ;
- une β-xylosidase, préférablement une β-xylosidase GH43 ou GH3, plus préférablement une β-xylosidase ayant au moins 55%, 60%, 65%, 70%, 75%, 80%, 85%, 90%, 95%, 99% ou 100% d'identité avec la séquence complète d'acide aminés présentée dans SEQ ID N°8 ou SEQ ID N°10 ;
- une glucanase, préférablement une glucanase GH8, plus préférablement une glucanase ayant au moins 55%, 60%, 65%, 70%, 75%, 80%, 85%, 90%, 95%, 99% ou 100% d'identité avec la séquence complète d'acide aminés présentée dans SEQ ID N°14 ou SEQ ID N°20 ;
- une arabinanase, préférablement une arabinanase GH43, plus préférablement une arabinanase ayant au moins 55%, 60%, 65%, 70%, 75%, 80%, 85%, 90%, 95%, 99% ou 100% d'identité avec la séquence complète d'acide aminés présentée dans SEQ ID N°12 ; et
- une estérase, préférablement une estérase CE1, plus préférablement une estérase ayant au moins 55%, 60%, 65%, 70%, 75%, 80%, 85%, 90%, 95%, 99% ou 100% d'identité avec la séquence complète d'acide aminés présentée dans SEQ ID N°16 ou SEQ ID N°18.


### SEQUENCE LISTING

<110> CARBIOS
<120> HEMICELLULOSE DEGRADING COMPOSITIONS AND USES THEREOF
<130> B1977PC00
<160> 40
<170> PatentIn version 3.5
<210> 1
   <211> 2379
   <212> DNA
   <213> artificial sequence
<220>
   <223> alpha-L-arabinofuranosidase GH3 (Abf_3)
<400> 1
   atgactcacc cgtatcagga tgcttcacaa cccgtcgcta cccgcgtagc cgatctgctc      60
   gcccgcatga cgccggagga aaagtttgcc cagatgcatg ccttctggct gatcctcgac     120
   agtcagggta aacacaaaga gcgcaccgat ctgagcgatg attttgccgg acataaacaa     180
   actttggcgc tggaggaaca actggcgctt ggtgcaggac aaatcacacg accactggga     240
   acacacgtgg tcagcccggc tgaaggcgtg cgcgcgctaa accgattgca aaaaacactg     300
   gtggaagaaa cgcggctggg cattccggcc atgttccacg aagaatgtct ggttggtctg     360
   ctttgcaaag acgcgacgct tttcccctct tctctgagct atgcctcgac gtgggatccg     420
   caactgatgg agcagattgc aggcgctatc cgccacgaag cgcaaagcgt cggttgtcat     480
   cagggactgg caccggtgct ggatgtgtcc cgtgatgtcc gctggggccg taccgaagaa     540
   acgtttggtg aggatcctta tctggtcggc gtgatggcaa ccgcttatgt acgcggtttg     600
   cagggcgaaa atcgtgatct gctggccacg cttaagcatt atgtcgggca ttcgttcagt     660
   gaaggcgcgc gcaatcatgc gccggtacat ctgggttttc gtgagctgaa tgacacgttc     720
   ctgctgcctt ttgaaatggc agtgaagctg gcgaatgccg gttcggtgat gcccgcttat     780
   catgatatcg acggtcagcc gtgtcacagc gatcacttcc tgctgactga aattttgcgt     840
   gaacaatggg gattcgatgg cctgattgtt gccgattacg gcggtgtaag tctgctgcat     900
   cagcatcacg gtgtcagcca tgacgatgcc gaatccgctg cactgtcgtt caatgccggg     960
   ctggatatcg aattaccgaa aggcgattgc gcacaacatc tggctgatgc ggtttcccgt    1020
   ggtctgattg atatggcaaa aattgatgag attgttgcgc gcgtgctgac cgaaaaattc    1080
   cgactcggtc tgtttgaaca gccttacgcc attgagccgc aaccgcaact gcaacaaccc    1140
   gccacgcgtg acctggcctg gaaagctgct gtccgctcca ttaccctgct ggaaaataat    1200
   ggcattcttc cgcttaagcc aaaatccggc cagaaaatcg ccgtcatcgg cccgacggca    1260
   gacgatccgc tggccttact cagcggttac agttttccgg tacatctgat catcagcgaa 1320
   atggaagcac agacggaaca ggtcattacg ccgttacagg ccttacgtac tgcatatggc 1380
   gaacaaaata ttcgttatgc caaaggctgt catattctcg aaaaacgcac ggcgggcgca 1440
   ccggttttcc ccggtgatgc cgcaacgccg atgcaaaact cgccggtatc actgagcacc 1500
   acgctcatcc cgcaggctgt aaaactggcg caggaaagcg acgtcgcgct ggtgtttgtc 1560
   ggcgatctcg ccggattatt ccagagcggc acagtcggcg aaggttctga taccgacagc 1620
   ctgcaactgc cgggcgtaca gcagcagttg ttggaaaaga ttgtagcgac agggaaaccg 1680
   accattgtgg tcatgaccgg cggtcgtccg tattcccttg gagggctgga ggacaaggtc 1740
   gccggacttc tgatggcgtt tgcgcccggt caggaaggcg gacgtgcaat tgccgatatt 1800
   gtcagcggca aagaagcacc ctccggacgg ctggtcgttt ccgtgccgaa aagcgccggt 1860
   gcaatgccgt attactacaa tcacaaactg aaaagtggcg gtacgccaat cgcgttccat 1920
   tttggtgcca gatacccgtt tggcttcggg ctgagcggca ccacgttccg ctatcacgat 1980
   ttacaactga atcagcagcc ggtcagcatt gaaaacggtg aaattacggt gcaaatcaaa 2040
   gtgaccaata ccggcagtca gacgggcagc gaagtggtac aagtttatct gcgcgactgc 2100
   gtggcatcac tcgtgcgacc ggttcaggaa ctcaaagctt ttgagcgggt tgaacttgaa 2160
   accggacata cggcgacgct cagtttcagt attcccaccg acatgctgaa tttcaccagt 2220
   cacgcgggta agcgcatcgt cgagccgggt tttgtcgagg tacaggtagg atcatcaagc 2280
   gcggatattc atgcaacggg caaagtgatt ctggcgggtg aagtacgcga attgccgcgc 2340
   aactggcgga tgctcagccg ttgtgaaatt agccaataa 2379
<210> 2
   <211> 792
   <212> PRT
   <213> artificial sequence
<220>
   <223> alpha-L-arabinofuranosidase GH3 (Abf_3)
<400> 2
   Met Thr His Pro Tyr Gln Asp Ala Ser Gln Pro Val Ala Thr Arg Val
   1               5                   10                  15
   Ala Asp Leu Leu Ala Arg Met Thr Pro Glu Glu Lys Phe Ala Gln Met
               20                  25                  30
   His Ala Phe Trp Leu Ile Leu Asp Ser Gln Gly Lys His Lys Glu Arg
           35                  40                  45
   Thr Asp Leu Ser Asp Asp Phe Ala Gly His Lys Gln Thr Leu Ala Leu
       50                  55                  60
   Glu Glu Gln Leu Ala Leu Gly Ala Gly Gln Ile Thr Arg Pro Leu Gly
   65                  70                  75                  80
   Thr His Val Val Ser Pro Ala Glu Gly Val Arg Ala Leu Asn Arg Leu
                   85                  90                  95
   Gln Lys Thr Leu Val Glu Glu Thr Arg Leu Gly Ile Pro Ala Met Phe
               100                 105                 110
   His Glu Glu Cys Leu Val Gly Leu Leu Cys Lys Asp Ala Thr Leu Phe
           115                 120                 125
   Pro Ser Ser Leu Ser Tyr Ala Ser Thr Trp Asp Pro Gln Leu Met Glu
       130                 135                 140
   Gln Ile Ala Gly Ala Ile Arg His Glu Ala Gln Ser Val Gly Cys His
   145                 150                 155                 160
   Gln Gly Leu Ala Pro Val Leu Asp Val Ser Arg Asp Val Arg Trp Gly
                   165                 170                 175
   Arg Thr Glu Glu Thr Phe Gly Glu Asp Pro Tyr Leu Val Gly Val Met
               180                 185                 190
   Ala Thr Ala Tyr Val Arg Gly Leu Gln Gly Glu Asn Arg Asp Leu Leu
           195                 200                 205
   Ala Thr Leu Lys His Tyr Val Gly His Ser Phe Ser Glu Gly Ala Arg
       210                 215                 220
   Asn His Ala Pro Val His Leu Gly Phe Arg Glu Leu Asn Asp Thr Phe
   225                 230                 235                 240
   Leu Leu Pro Phe Glu Met Ala Val Lys Leu Ala Asn Ala Gly Ser Val
                   245                 250                 255
   Met Pro Ala Tyr His Asp Ile Asp Gly Gln Pro Cys His Ser Asp His
               260                 265                 270
   Phe Leu Leu Thr Glu Ile Leu Arg Glu Gln Trp Gly Phe Asp Gly Leu
           275                 280                 285
   Ile Val Ala Asp Tyr Gly Gly Val Ser Leu Leu His Gln His His Gly
       290                 295                 300
   Val Ser His Asp Asp Ala Glu Ser Ala Ala Leu Ser Phe Asn Ala Gly
   305                 310                 315                 320
   Leu Asp Ile Glu Leu Pro Lys Gly Asp Cys Ala Gln His Leu Ala Asp
                   325                 330                 335
   Ala Val Ser Arg Gly Leu Ile Asp Met Ala Lys Ile Asp Glu Ile Val
               340                 345                 350
   Ala Arg Val Leu Thr Glu Lys Phe Arg Leu Gly Leu Phe Glu Gln Pro
           355                 360                 365
   Tyr Ala Ile Glu Pro Gln Pro Gln Leu Gln Gln Pro Ala Thr Arg Asp
       370                 375                 380
   Leu Ala Trp Lys Ala Ala Val Arg Ser Ile Thr Leu Leu Glu Asn Asn
   385                 390                 395                 400
   Gly Ile Leu Pro Leu Lys Pro Lys Ser Gly Gln Lys Ile Ala Val Ile
                   405                 410                 415
   Gly Pro Thr Ala Asp Asp Pro Leu Ala Leu Leu Ser Gly Tyr Ser Phe
               420                 425                 430
   Pro Val His Leu Ile Ile Ser Glu Met Glu Ala Gln Thr Glu Gln Val
           435                 440                 445
   Ile Thr Pro Leu Gln Ala Leu Arg Thr Ala Tyr Gly Glu Gln Asn Ile
       450                 455                 460
   Arg Tyr Ala Lys Gly Cys His Ile Leu Glu Lys Arg Thr Ala Gly Ala
   465                 470                 475                 480
   Pro Val Phe Pro Gly Asp Ala Ala Thr Pro Met Gln Asn Ser Pro Val
                   485                 490                 495
   Ser Leu Ser Thr Thr Leu Ile Pro Gln Ala Val Lys Leu Ala Gln Glu
               500                 505                 510
   Ser Asp Val Ala Leu Val Phe Val Gly Asp Leu Ala Gly Leu Phe Gln
           515                 520                 525
   Ser Gly Thr Val Gly Glu Gly Ser Asp Thr Asp Ser Leu Gln Leu Pro
       530                 535                 540
   Gly Val Gln Gln Gln Leu Leu Glu Lys Ile Val Ala Thr Gly Lys Pro
   545                 550                 555                 560
   Thr Ile Val Val Met Thr Gly Gly Arg Pro Tyr Ser Leu Gly Gly Leu
   565 570 575
   Glu Asp Lys Val Ala Gly Leu Leu Met Ala Phe Ala Pro Gly Gln Glu
   580 585 590
   Gly Gly Arg Ala Ile Ala Asp Ile Val Ser Gly Lys Glu Ala Pro Ser
   595 600 605
   Gly Arg Leu Val Val Ser Val Pro Lys Ser Ala Gly Ala Met Pro Tyr
   610 615 620
   Tyr Tyr Asn His Lys Leu Lys Ser Gly Gly Thr Pro Ile Ala Phe His
   625 630 635 640
   Phe Gly Ala Arg Tyr Pro Phe Gly Phe Gly Leu Ser Gly Thr Thr Phe
   645 650 655
   Arg Tyr His Asp Leu Gln Leu Asn Gln Gln Pro Val Ser Ile Glu Asn
   660 665 670
   Gly Glu Ile Thr Val Gln Ile Lys Val Thr Asn Thr Gly Ser Gln Thr
   675 680 685
   Gly Ser Glu Val Val Gln Val Tyr Leu Arg Asp Cys Val Ala Ser Leu
   690 695 700
   Val Arg Pro Val Gln Glu Leu Lys Ala Phe Glu Arg Val Glu Leu Glu
   705 710 715 720
   Thr Gly His Thr Ala Thr Leu Ser Phe Ser Ile Pro Thr Asp Met Leu
   725 730 735
   Asn Phe Thr Ser His Ala Gly Lys Arg Ile Val Glu Pro Gly Phe Val
   740 745 750
   Glu Val Gln Val Gly Ser Ser Ser Ala Asp Ile His Ala Thr Gly Lys
   755 760 765
   Val Ile Leu Ala Gly Glu Val Arg Glu Leu Pro Arg Asn Trp Arg Met
   770 775 780
   Leu Ser Arg Cys Glu Ile Ser Gln
   785 790
<210> 3
   <211> 678
   <212> DNA
   <213> artificial sequence
<220>
   <223> xylanase GH 11 (Np-Xyn)
<400> 3
   atgaagttta ctgtcggtaa tggacaaaac caacataagg gtgtcaacga tggtttcagt    60
   tatgaaatct ggttagataa cactggtggt aacggttcta tgactctcgg tagtggtgca   120
   actttcaagg ctgaatggaa tgcagctgtt aaccgtggta acttccttgc ccgtcgtggt   180
   cttgacttcg gttctcaaaa gaaggcaacc gattacgact acattggatt agattatgct   240
   gctacttaca aacaaactgc cagtgcaagt ggtaactccc gtctctgtgt atacggatgg   300
   ttccaaaacc gtggacttaa tggcgttcct ttagtagaat actacatcat tgaagattgg   360
   gttgactggg ttccagatgc acaaggaaaa atggtaacca ttgatggagc tcaatataag   420
   attttccaaa tggatcacac tggtccaact atcaatggtg gtagtgaaac ctttaagcaa   480
   tacttcagtg tccgtcaaca aaagagaact tctggtcata ttactgtctc agatcacttt   540
   aaggaatggg ccaaacaagg ttggggtatt ggtaaccttt atgaagtggc tttgaacgcc   600
   gaaggttggc aaagtagtgg tgttgctgat gtcaccttat tagatgttta cacaactcca   660
   aagggttcta gtccagcc                                                 678
<210> 4
   <211> 224
   <212> PRT
   <213> artificial sequence
<220>
   <223> xylanase GH 11 (Np-Xyn)
<400> 4
   Met Lys Phe Thr Val Gly Asn Gly Gln Asn Gln His Lys Gly Val Asn
   1               5                   10                  15
   Asp Gly Phe Ser Tyr Glu Ile Trp Leu Asp Asn Thr Gly Gly Asn Gly
               20                  25                  30
   Ser Met Thr Leu Gly Ser Gly Ala Thr Phe Lys Ala Glu Trp Asn Ala
           35                  40                  45
   Ala Val Asn Arg Gly Asn Phe Leu Ala Arg Arg Gly Leu Asp Phe Gly
       50                  55                  60
   Ser Gln Lys Lys Ala Thr Asp Tyr Asp Tyr Ile Gly Leu Asp Tyr Ala
   65                  70                  75                  80
   Ala Thr Tyr Lys Gln Thr Ala Ser Ala Ser Gly Asn Ser Arg Leu Cys
                   85                  90                  95
   Val Tyr Gly Trp Phe Gln Asn Arg Gly Leu Asn Gly Val Pro Leu Val
               100                 105                 110
   Glu Tyr Tyr Ile Ile Glu Asp Trp Val Asp Trp Val Pro Asp Ala Gln
           115                 120                 125
   Gly Lys Met Val Thr Ile Asp Gly Ala Gln Tyr Lys Ile Phe Gln Met
       130                 135                 140
   Asp His Thr Gly Pro Thr Ile Asn Gly Gly Ser Glu Thr Phe Lys Gln
   145                 150                 155                 160
   Tyr Phe Ser Val Arg Gln Gln Lys Arg Thr Ser Gly His Ile Thr Val
                   165                 170                 175
   Ser Asp His Phe Lys Glu Trp Ala Lys Gln Gly Trp Gly Ile Gly Asn
               180                 185                 190
   Leu Tyr Glu Val Ala Leu Asn Ala Glu Gly Trp Gln Ser Ser Gly Val
           195                 200                 205
   Ala Asp Val Thr Leu Leu Asp Val Tyr Thr Thr Pro Lys Gly Ser Ser
       210                 215                 220
<210> 5
   <211> 2235
   <212> DNA
   <213> artificial sequence
<220>
   <223> xylanase GH 10 (Xyn_10)
<400> 5
   gaaaaaatga agtgggacga tccctatacc cacataccga cgggagacct gccgatcgaa      60
   ctacaggaaa agatcgcgat gtatgaagcg atcaacgctt acgccacttt caagatcggc     120
   gtggccgttg atttcggcct ctacatgaca gatgaggagt accgcgccat cgtggacgag     180
   aatttcgacg aagtggtacc cggcaacgaa atgaagcagc agagcctgat gaacgcccag     240
   ggagttctga atttcgacag gaacgacccc acgatcgaag agttgctggc cgccggcctg     300
   gacatttacg ggcataccct cgtgtggtat cagcaggcgc aggcctccta tttcaacagc     360
   ctgatagcac ccactatcgt tcccgggact ccgggttcga gccttgtgga cggaagtttc     420
   gaagagggga tgggcggctg gacacccgct ttcaacgcgc agaattacac ggtcgtggac     480
   accgaggcgg tcgacgggac gcaatccctg caggtggtcg tcagcggctc gaccggaaaa     540
   tacgacgcac aactcaactc tccggcgttc ccgatcgtcg cgggacacca ttaccagctc     600
   tctttctgga tcaagggctc cgtccccgga aaggtgggga tcgacttccc gaacgcggaa 660
   ctgggtaatc aatatccatg ggtgaacggc gcagaactgg ccgacgtggg aacctcctgg 720
   acccaggtga tctacaacac cgaaaccgtg ggcgacccgg cgatgatcgc cacagccgac 780
   aacggcgcga tgacggtacg gttacttctg ggcggtgtcg cggagtgtac atatctgatc 840
   gacgccgtcg agatcacaga cctcgacgat gcgcccgtgg acaccaacct tatctcggac 900
   ggcaatttcg aaggcggagc catgggagac tgggtcgcac cctattattc cgacgaatac 960
   acggtggtcg acaccgaatc gttcgacggc acccactcgt tgcaggtcgc cgtgggcagc 1020
   ggtgcggctg gcaaatacga ccgccaactc aacagcccgg catttccgat cataacggga 1080
   caccattacc agatctcctt ctggatcaag agcaacggcg acggaaaggt cggcctcgac 1140
   tttccgtcga acgaactcgg caaccagtat ccgtggggaa gcggcggcga gttggccacc 1200
   gtcggcacga gctggacgca agttacctac aacaccaccg tttcgggcga cccggcaatg 1260
   atcgccaccg ccgataacgc cgcgatgacc ttccgcctgt tgctcggcgc ggtcgcggac 1320
   tgcacctact ggatcgacgc cgttgaggtc accgacctcg acaccgcgaa cgccggggcg 1380
   cgcgtggctc cccggatgat gaccagggct tccggcccca taaccatcga aaagaccccc 1440
   gaagagaaaa aggagatcat cggagcggcg ttcgtcaagt acatcaccga cgtggccacc 1500
   cattacaaag gcaaggtgac ggcgtgggat gtgctgaacg aacccatgaa cgagaacggc 1560
   acgctgaggg tcggtacgga agacctctcg gcgacgagcg ttttccactg ggcttactat 1620
   ctgggcgaag attacgccgt gaccgccttc aaaacggcac gcgaagcaga ccccgatgcg 1680
   aaactgttca tcaacgacta cggtctcgaa tcgacgggcg gcgctaaact cgacggcctc 1740
   atcggatatg tgaaccatat cgaagccaac ggcggacagg tcgacggcat cggcacgcaa 1800
   atgcacctca acctcaactg ggtggacagg gacgccatca ccgccatgtt caccaaactg 1860
   gccgcgacgg gcaaactcat caaggtgacg gagctcgata ttgccatatc caccgaatcg 1920
   agcgcgggtg gaggccccgc aacgcccctt tcgccggcgg cggcggaact ggaagcacag 1980
   gccgacctgt atggattcgt ggcgaaaacg tactccgaga tcatcccgcc ggcgcagcaa 2040
   tatggcatta ccctgtgggg cgtttcggac aatcccgagc agcacctata ctggctgccc 2100
   aacgacgcgc cgtgcctgtg ggacgccgat tatcagcgca aatgggcgta taagggcttc 2160
   tgcgacggtt tgtatggtag cgacgtgagc gccgactgga catacgacga ccttgtgaac 2220
   gacgcagcaa agtaa 2235
<210> 6
   <211> 744
   <212> PRT
   <213> artificial sequence
<220>
   <223> xylanase GH 10 (Xyn_10)
<400> 6
   Glu Lys Met Lys Trp Asp Asp Pro Tyr Thr His Ile Pro Thr Gly Asp
   1               5                   10                  15
   Leu Pro Ile Glu Leu Gln Glu Lys Ile Ala Met Tyr Glu Ala Ile Asn
               20                  25                  30
   Ala Tyr Ala Thr Phe Lys Ile Gly Val Ala Val Asp Phe Gly Leu Tyr
           35                  40                  45
   Met Thr Asp Glu Glu Tyr Arg Ala Ile Val Asp Glu Asn Phe Asp Glu
       50                  55                  60
   Val Val Pro Gly Asn Glu Met Lys Gln Gln Ser Leu Met Asn Ala Gln
   65                  70                  75                  80
   Gly Val Leu Asn Phe Asp Arg Asn Asp Pro Thr Ile Glu Glu Leu Leu
                   85                  90                  95
   Ala Ala Gly Leu Asp Ile Tyr Gly His Thr Leu Val Trp Tyr Gln Gln
               100                 105                 110
   Ala Gln Ala Ser Tyr Phe Asn Ser Leu Ile Ala Pro Thr Ile Val Pro
           115                 120                 125
   Gly Thr Pro Gly Ser Ser Leu Val Asp Gly Ser Phe Glu Glu Gly Met
       130                 135                 140
   Gly Gly Trp Thr Pro Ala Phe Asn Ala Gln Asn Tyr Thr Val Val Asp
   145                 150                 155                 160
   Thr Glu Ala Val Asp Gly Thr Gln Ser Leu Gln Val Val Val Ser Gly
                   165                 170                 175
   Ser Thr Gly Lys Tyr Asp Ala Gln Leu Asn Ser Pro Ala Phe Pro Ile
               180                 185                 190
   Val Ala Gly His His Tyr Gln Leu Ser Phe Trp Ile Lys Gly Ser Val
           195                 200                 205
   Pro Gly Lys Val Gly Ile Asp Phe Pro Asn Ala Glu Leu Gly Asn Gln
       210                 215                 220
   Tyr Pro Trp Val Asn Gly Ala Glu Leu Ala Asp Val Gly Thr Ser Trp
   225                 230                 235                 240
   Thr Gln Val Ile Tyr Asn Thr Glu Thr Val Gly Asp Pro Ala Met Ile
   245 250 255
   Ala Thr Ala Asp Asn Gly Ala Met Thr Val Arg Leu Leu Leu Gly Gly
   260 265 270
   Val Ala Glu Cys Thr Tyr Leu Ile Asp Ala Val Glu Ile Thr Asp Leu
   275 280 285
   Asp Asp Ala Pro Val Asp Thr Asn Leu Ile Ser Asp Gly Asn Phe Glu
   290 295 300
   Gly Gly Ala Met Gly Asp Trp Val Ala Pro Tyr Tyr Ser Asp Glu Tyr
   305 310 315 320
   Thr Val Val Asp Thr Glu Ser Phe Asp Gly Thr His Ser Leu Gln Val
   325 330 335
   Ala Val Gly Ser Gly Ala Ala Gly Lys Tyr Asp Arg Gln Leu Asn Ser
   340 345 350
   Pro Ala Phe Pro Ile Ile Thr Gly His His Tyr Gln Ile Ser Phe Trp
   355 360 365
   Ile Lys Ser Asn Gly Asp Gly Lys Val Gly Leu Asp Phe Pro Ser Asn
   370 375 380
   Glu Leu Gly Asn Gln Tyr Pro Trp Gly Ser Gly Gly Glu Leu Ala Thr
   385 390 395 400
   Val Gly Thr Ser Trp Thr Gln Val Thr Tyr Asn Thr Thr Val Ser Gly
   405 410 415
   Asp Pro Ala Met Ile Ala Thr Ala Asp Asn Ala Ala Met Thr Phe Arg
   420 425 430
   Leu Leu Leu Gly Ala Val Ala Asp Cys Thr Tyr Trp Ile Asp Ala Val
   435 440 445
   Glu Val Thr Asp Leu Asp Thr Ala Asn Ala Gly Ala Arg Val Ala Pro
   450 455 460
   Arg Met Met Thr Arg Ala Ser Gly Pro Ile Thr Ile Glu Lys Thr Pro
   465 470 475 480
   Glu Glu Lys Lys Glu Ile Ile Gly Ala Ala Phe Val Lys Tyr Ile Thr
   485 490 495
   Asp Val Ala Thr His Tyr Lys Gly Lys Val Thr Ala Trp Asp Val Leu
   500 505 510
   Asn Glu Pro Met Asn Glu Asn Gly Thr Leu Arg Val Gly Thr Glu Asp
   515 520 525
   Leu Ser Ala Thr Ser Val Phe His Trp Ala Tyr Tyr Leu Gly Glu Asp
   530 535 540
   Tyr Ala Val Thr Ala Phe Lys Thr Ala Arg Glu Ala Asp Pro Asp Ala
   545 550 555 560
   Lys Leu Phe Ile Asn Asp Tyr Gly Leu Glu Ser Thr Gly Gly Ala Lys
   565 570 575
   Leu Asp Gly Leu Ile Gly Tyr Val Asn His Ile Glu Ala Asn Gly Gly
   580 585 590
   Gln Val Asp Gly Ile Gly Thr Gln Met His Leu Asn Leu Asn Trp Val
   595 600 605
   Asp Arg Asp Ala Ile Thr Ala Met Phe Thr Lys Leu Ala Ala Thr Gly
   610 615 620
   Lys Leu Ile Lys Val Thr Glu Leu Asp Ile Ala Ile Ser Thr Glu Ser
   625 630 635 640
   Ser Ala Gly Gly Gly Pro Ala Thr Pro Leu Ser Pro Ala Ala Ala Glu
   645 650 655
   Leu Glu Ala Gln Ala Asp Leu Tyr Gly Phe Val Ala Lys Thr Tyr Ser
   660 665 670
   Glu Ile Ile Pro Pro Ala Gln Gln Tyr Gly Ile Thr Leu Trp Gly Val
   675 680 685
   Ser Asp Asn Pro Glu Gln His Leu Tyr Trp Leu Pro Asn Asp Ala Pro
   690 695 700
   Cys Leu Trp Asp Ala Asp Tyr Gln Arg Lys Trp Ala Tyr Lys Gly Phe
   705 710 715 720
   Cys Asp Gly Leu Tyr Gly Ser Asp Val Ser Ala Asp Trp Thr Tyr Asp
   725 730 735
   Asp Leu Val Asn Asp Ala Ala Lys
   740
<210> 7
   <211> 1587
   <212> DNA
   <213> artificial sequence
<220>
   <223> xylosidase GH 43 (Bh-Xyl)
<400> 7
   atggtcaatc gtatccaaaa tcctattttg ccagggtttc atccagaccc atccattgtc 60
   cgtgttggtg atgattacta tatcgccacc tctacatttg aatggtttcc tggggtgcgc 120
   atccaccatt ctcgggattt aaaacattgg cgctttgtat ctagtccgct gacccgcact 180
   tcccaactag acatgaaagg gaatatgaac tccggcggga tatgggcgcc atgcctaagc 240
   tatcatgacg gaacctttta tttgatctat actgatgtga agcaatggca cggtgccttc 300
   aaagacgcgc acaactattt agtgacggca caaaacattg aagggccgtg gtcggacccg 360
   atttacttaa acagtagcgg ctttgacccg tccctgtttc acgatgacga tggccgaaaa 420
   tggctcgtta acatgatctg ggactaccgc aaaggaaacc atccttttgc cggaattatt 480
   ttgcaagaat actcagaagc agaacaaaaa cttgtcgggc ctgtgaaaaa tatctataaa 540
   gggaccgaca ttcagctaac agagggaccg cacctctata agaaagatgg ttattattat 600
   ttacttgttg ccgaaggagg gacggaatac gaacacgccg cgaccctcgc ccgctcacag 660
   tcaattgacg gaccctatga gaccgacccg agttatccac tcgtcacatc gactggccag 720
   ccggaattgg cgttgcaaaa ggccggacac ggtagcctcg tagaaaccca gaacggcgaa 780
   tggtatctcg ctcacttgtg cggtcgccca ttaaaaggaa agtactgcac actcggcagg 840
   gaaacagcca ttcaaaaagt aaactggacc gaggatggct ggctgcgcat cgaggatggc 900
   ggcaatcacc cgttgcgtga agtgacggca cctgaccttc cagagcaccc attcgaaaaa 960
   gaacccgagc tcgatgattt tgacgcaccc cagctgcacc atcaatggaa cacgctgcgc 1020
   atccctgccg acccatcatg gtgctcgctc gaggaacgtc cgggccattt acgactgcgc 1080
   gggatggagt ccctcacttc cgtccactcg caaagtttag tcgcccgcag gcagcagtcc 1140
   ttccactgcg aagttgagac aaagctagag tatcagccag aatcgtttca acatatggct 1200
   gggcttgtca tttactatga cacagaagat catgtctatt tgcacgtaac ctggcacgag 1260
   gaaaagggta aatgtctaca aatcatacag acaaagggcg gaaactatga cgaattgctt 1320
   gcgtcaccga tcccactggc agaagaaaag gcggtttatt tgaaggggcg cattcaccgt 1380
   gaaacgatgc acctctattt caaacaagag ggagaagcgg aatggcagcc tgtggggcca 1440
   acgattgatg tgacccacat gtccgacgat tcagcgaagc aagttcgatt taccggcaca 1500
   tttgtcggca tggctacgca agacttgagc ggaacgaaaa agccagccga ttttgattac 1560
   tttcgctata aagaactaga tcaataa 1587
<210> 8
   <211> 528
   <212> PRT
   <213> artificial sequence
<220>
   <223> xylosidase GH 43 (Bh-Xyl)
<400> 8
   Met Val Asn Arg Ile Gln Asn Pro Ile Leu Pro Gly Phe His Pro Asp
   1               5                   10                  15
   Pro Ser Ile Val Arg Val Gly Asp Asp Tyr Tyr Ile Ala Thr Ser Thr
               20                  25                  30
   Phe Glu Trp Phe Pro Gly Val Arg Ile His His Ser Arg Asp Leu Lys
           35                  40                  45
   His Trp Arg Phe Val Ser Ser Pro Leu Thr Arg Thr Ser Gln Leu Asp
       50                  55                  60
   Met Lys Gly Asn Met Asn Ser Gly Gly Ile Trp Ala Pro Cys Leu Ser
   65                  70                  75                  80
   Tyr His Asp Gly Thr Phe Tyr Leu Ile Tyr Thr Asp Val Lys Gln Trp
                   85                  90                  95
   His Gly Ala Phe Lys Asp Ala His Asn Tyr Leu Val Thr Ala Gln Asn
               100                 105                 110
   Ile Glu Gly Pro Trp Ser Asp Pro Ile Tyr Leu Asn Ser Ser Gly Phe
           115                 120                 125
   Asp Pro Ser Leu Phe His Asp Asp Asp Gly Arg Lys Trp Leu Val Asn
       130                 135                 140
   Met Ile Trp Asp Tyr Arg Lys Gly Asn His Pro Phe Ala Gly Ile Ile
   145                 150                 155                 160
   Leu Gln Glu Tyr Ser Glu Ala Glu Gln Lys Leu Val Gly Pro Val Lys
                   165                 170                 175
   Asn Ile Tyr Lys Gly Thr Asp Ile Gln Leu Thr Glu Gly Pro His Leu
               180                 185                 190
   Tyr Lys Lys Asp Gly Tyr Tyr Tyr Leu Leu Val Ala Glu Gly Gly Thr
           195                 200                 205
   Glu Tyr Glu His Ala Ala Thr Leu Ala Arg Ser Gln Ser Ile Asp Gly
       210                 215                 220
   Pro Tyr Glu Thr Asp Pro Ser Tyr Pro Leu Val Thr Ser Thr Gly Gln
   225                 230                 235                 240
   Pro Glu Leu Ala Leu Gln Lys Ala Gly His Gly Ser Leu Val Glu Thr
                   245                 250                 255
   Gln Asn Gly Glu Trp Tyr Leu Ala His Leu Cys Gly Arg Pro Leu Lys
               260                 265                 270
   Gly Lys Tyr Cys Thr Leu Gly Arg Glu Thr Ala Ile Gln Lys Val Asn
           275                 280                 285
   Trp Thr Glu Asp Gly Trp Leu Arg Ile Glu Asp Gly Gly Asn His Pro
       290                 295                 300
   Leu Arg Glu Val Thr Ala Pro Asp Leu Pro Glu His Pro Phe Glu Lys
   305                 310                 315                 320
   Glu Pro Glu Leu Asp Asp Phe Asp Ala Pro Gln Leu His His Gln Trp
                   325                 330                 335
   Asn Thr Leu Arg Ile Pro Ala Asp Pro Ser Trp Cys Ser Leu Glu Glu
               340                 345                 350
   Arg Pro Gly His Leu Arg Leu Arg Gly Met Glu Ser Leu Thr Ser Val
           355                 360                 365
   His Ser Gln Ser Leu Val Ala Arg Arg Gln Gln Ser Phe His Cys Glu
       370                 375                 380
   Val Glu Thr Lys Leu Glu Tyr Gln Pro Glu Ser Phe Gln His Met Ala
   385                 390                 395                 400
   Gly Leu Val Ile Tyr Tyr Asp Thr Glu Asp His Val Tyr Leu His Val
                   405                 410                 415
   Thr Trp His Glu Glu Lys Gly Lys Cys Leu Gln Ile Ile Gln Thr Lys
               420                 425                 430
   Gly Gly Asn Tyr Asp Glu Leu Leu Ala Ser Pro Ile Pro Leu Ala Glu
           435                 440                 445
   Glu Lys Ala Val Tyr Leu Lys Gly Arg Ile His Arg Glu Thr Met His
       450                 455                 460
   Leu Tyr Phe Lys Gln Glu Gly Glu Ala Glu Trp Gln Pro Val Gly Pro
   465                 470                 475                 480
   Thr Ile Asp Val Thr His Met Ser Asp Asp Ser Ala Lys Gln Val Arg
                   485                 490                 495
   Phe Thr Gly Thr Phe Val Gly Met Ala Thr Gln Asp Leu Ser Gly Thr
               500                 505                 510
   Lys Lys Pro Ala Asp Phe Asp Tyr Phe Arg Tyr Lys Glu Leu Asp Gln
           515                 520                 525
<210> 9
   <211> 2289
   <212> DNA
   <213> artificial sequence
<220>
   <223> beta-xylosidase GH 3 (Xyl_3)
<400> 9
   caggaagcgc cccaacttcg cgccgacaac atagacgaag tgatggcggc gatgaccctc       60
   gatgaaaaag tgacgctgct gataggcacc gggatggccg gtttcgacgg ctcgaacacg      120
   gtcatcggag ccacccagaa cctcgtgccg ggagcggcgg gcacatccaa ccccattccg      180
   aggctgggta ttcccgccat cgtgctggcc gacgggcctg ccggattgcg catcgccccc      240
   acccgcaaag gcgacaagaa cacctattac tgcaccggct tccccatcgc gacggtgatg      300
   ggcagcacct ggaacaccgc cctggtggaa gaagtggggc gtgctatggg taacgaagtg      360
   ctggaatacg gcgcggacgt gcttttggcc cccgcgctga acctgcaccg caatccgttg      420
   tgcggccgta acttcgagta ctattcggag gaccccctgc tggcgggaaa gatcaccgcg      480
   gcgatggtgc gtggcattca gggcaacggc gtgggcacct cgatcaagca cttcgcggcg      540
   aacaaccagg agaccaaccg taacgccaac gacgcgcgta tgacgacccg cacccttcgc      600
   gagatgtacc tgcgggggtt cgagatcgcg gtgaaggaat cggcgccctg gacggtgatg      660
   tcgtcgtaca acaaggtaaa cggcacgtac actcccgaga gtcgcgatct gctcaccgac      720
   attctgcgcg gcgagtgggg cttcgaaggt cttgtgatga ccgactggtt cggaggtaag      780
   gacgcagcag cgacggttca cgccggcaac gaccttatga tgcccggaca ccccggccag      840
   aagcaggtga taatggcggc actggagaac ggatcgctgg acatcgccga catcgacacg      900
   aatgtgcgcc ggatgctgga actgatcgtt cgcacaccgc gtttcaaggg ttataaatac      960
   tccaacaagc ccgacctgac cgcacacgcc gcagtcaccc gctcctcggc tgccgaagga     1020
   atggtgttgc tgaagaacga cggcgacgcg ctgcctttag ccgccggggt gaaggaaatt 1080
   tcgctgttcg gcatcacttc ctatgacttt attgcgggcg gaaccggcag cggcgacgtg 1140
   aacaaggcct atgtggtgtc gctcgaacag ggcctgctga gcgccggtta tgtcattaat 1200
   gccgacggac gccggatgta tgccgatcat atcgccgccg agaaggcgaa agataaaccc 1260
   ggcgacaacc cgatgatggc gttcttcaac aaaaagcgca ttcccgagac cgttcccgca 1320
   ggccattttc tggaggaaca ggctaaggct tccgacgcgg cggtgatcac catcggccgc 1380
   agttcgggtg aatttgtaga ccgcaaactg gaaggcgact tcctgttgac ggccgacgag 1440
   atcgcgctga tgaaagaggt gaaggcggcg ttcgccaaag agggcaagaa gaccctcgtc 1500
   atcctcaaca tcggcggcgt ggtggagacc gcgagctgga aagatattcc cgacgccatt 1560
   ctccttgcct ggcagccggg tcaggagggc gggaacaccg tcgcagatat tctgaagggc 1620
   gccgtgaacc cctcgggcaa gctcccgatg acattcccgg tgaactattt cgacgctccc 1680
   tcgtcggcca acttcccgtg ggattacaag ttcgagttct cgatgggtat gttcacgggc 1740
   ggcgacccca agggcgacga acccgaagtg cgcaacgtcg attataccgt atatgaagag 1800
   ggtatctatg tgggttaccg cttcttcaac accttcggca agcaggtttc gtatccgttc 1860
   ggttacggac tttcgtacac gaccttcgaa tactccggtt tcgcggtcgc tgcggccggc 1920
   gacggatata acgtttcggt gaatgtgaaa aacaccggtc gcgcggcggg taaagaggtc 1980
   gtacagctgt atgtcggcgc acctgcggcg ggattcaaca cgagggcgac cgaagagttg 2040
   ctggcatttg ccaagacggc cgaactcgct cccggagcct cggaaacggt ggttatgcac 2100
   ttctccgccg aagacctgcg ccgttacgac gggcagcgct cggcctgggt ggtcgacaag 2160
   ggtgtttaca aactgatggt gggagcctct tcgcgtgacc tgcgcgggtt cctgtcgctg 2220
   gatattccgc acgagatcgt cgttcttcag acggagaacc tgatggcgcc cgccgaacag 2280
   atcaccgaa 2289
<210> 10
   <211> 768
   <212> PRT
   <213> artificial sequence
<220>
   <223> beta-xylosidase GH 3 (Xyl_3)
<400> 10
   Gln Glu Ala Pro Gln Leu Arg Ala Asp Asn Ile Asp Glu Val Met Ala
   1               5                   10                  15
   Ala Met Thr Leu Asp Glu Lys Val Thr Leu Leu Ile Gly Thr Gly Met
               20                  25                  30
   Ala Gly Phe Asp Gly Ser Asn Thr Val Ile Gly Ala Thr Gln Asn Leu
           35                  40                  45
   Val Pro Gly Ala Ala Gly Thr Ser Asn Pro Ile Pro Arg Leu Gly Ile
       50                  55                  60
   Pro Ala Ile Val Leu Ala Asp Gly Pro Ala Gly Leu Arg Ile Ala Pro
   65                  70                  75                  80
   Thr Arg Lys Gly Asp Lys Asn Thr Tyr Tyr Cys Thr Gly Phe Pro Ile
                   85                  90                  95
   Ala Thr Val Met Gly Ser Thr Trp Asn Thr Ala Leu Val Glu Glu Val
               100                 105                 110
   Gly Arg Ala Met Gly Asn Glu Val Leu Glu Tyr Gly Ala Asp Val Leu
           115                 120                 125
   Leu Ala Pro Ala Leu Asn Leu His Arg Asn Pro Leu Cys Gly Arg Asn
       130                 135                 140
   Phe Glu Tyr Tyr Ser Glu Asp Pro Leu Leu Ala Gly Lys Ile Thr Ala
   145                 150                 155                 160
   Ala Met Val Arg Gly Ile Gln Gly Asn Gly Val Gly Thr Ser Ile Lys
                   165                 170                 175
   His Phe Ala Ala Asn Asn Gln Glu Thr Asn Arg Asn Ala Asn Asp Ala
               180                 185                 190
   Arg Met Thr Thr Arg Thr Leu Arg Glu Met Tyr Leu Arg Gly Phe Glu
           195                 200                 205
   Ile Ala Val Lys Glu Ser Ala Pro Trp Thr Val Met Ser Ser Tyr Asn
       210                 215                 220
   Lys Val Asn Gly Thr Tyr Thr Pro Glu Ser Arg Asp Leu Leu Thr Asp
   225                 230                 235                 240
   Ile Leu Arg Gly Glu Trp Gly Phe Glu Gly Leu Val Met Thr Asp Trp
                   245                 250                 255
   Phe Gly Gly Lys Asp Ala Ala Ala Thr Val His Ala Gly Asn Asp Leu
               260                 265                 270
   Met Met Pro Gly His Pro Gly Gln Lys Gln Val Ile Met Ala Ala Leu
           275                 280                 285
   Glu Asn Gly Ser Leu Asp Ile Ala Asp Ile Asp Thr Asn Val Arg Arg
   290 295 300
   Met Leu Glu Leu Ile Val Arg Thr Pro Arg Phe Lys Gly Tyr Lys Tyr
   305 310 315 320
   Ser Asn Lys Pro Asp Leu Thr Ala His Ala Ala Val Thr Arg Ser Ser
   325 330 335
   Ala Ala Glu Gly Met Val Leu Leu Lys Asn Asp Gly Asp Ala Leu Pro
   340 345 350
   Leu Ala Ala Gly Val Lys Glu Ile Ser Leu Phe Gly Ile Thr Ser Tyr
   355 360 365
   Asp Phe Ile Ala Gly Gly Thr Gly Ser Gly Asp Val Asn Lys Ala Tyr
   370 375 380
   Val Val Ser Leu Glu Gln Gly Leu Leu Ser Ala Gly Tyr Val Ile Asn
   385 390 395 400
   Ala Asp Gly Arg Arg Met Tyr Ala Asp His Ile Ala Ala Glu Lys Ala
   405 410 415
   Lys Asp Lys Pro Gly Asp Asn Pro Met Met Ala Phe Phe Asn Lys Lys
   420 425 430
   Arg Ile Pro Glu Thr Val Pro Ala Gly His Phe Leu Glu Glu Gln Ala
   435 440 445
   Lys Ala Ser Asp Ala Ala Val Ile Thr Ile Gly Arg Ser Ser Gly Glu
   450 455 460
   Phe Val Asp Arg Lys Leu Glu Gly Asp Phe Leu Leu Thr Ala Asp Glu
   465 470 475 480
   Ile Ala Leu Met Lys Glu Val Lys Ala Ala Phe Ala Lys Glu Gly Lys
   485 490 495
   Lys Thr Leu Val Ile Leu Asn Ile Gly Gly Val Val Glu Thr Ala Ser
   500 505 510
   Trp Lys Asp Ile Pro Asp Ala Ile Leu Leu Ala Trp Gln Pro Gly Gln
   515 520 525
   Glu Gly Gly Asn Thr Val Ala Asp Ile Leu Lys Gly Ala Val Asn Pro
   530 535 540
   Ser Gly Lys Leu Pro Met Thr Phe Pro Val Asn Tyr Phe Asp Ala Pro
   545                 550                 555                 560
   Ser Ser Ala Asn Phe Pro Trp Asp Tyr Lys Phe Glu Phe Ser Met Gly
                   565                 570                 575
   Met Phe Thr Gly Gly Asp Pro Lys Gly Asp Glu Pro Glu Val Arg Asn
               580                 585                 590
   Val Asp Tyr Thr Val Tyr Glu Glu Gly Ile Tyr Val Gly Tyr Arg Phe
           595                 600                 605
   Phe Asn Thr Phe Gly Lys Gln Val Ser Tyr Pro Phe Gly Tyr Gly Leu
       610                 615                 620
   Ser Tyr Thr Thr Phe Glu Tyr Ser Gly Phe Ala Val Ala Ala Ala Gly
   625                 630                 635                 640
   Asp Gly Tyr Asn Val Ser Val Asn Val Lys Asn Thr Gly Arg Ala Ala
                   645                 650                 655
   Gly Lys Glu Val Val Gln Leu Tyr Val Gly Ala Pro Ala Ala Gly Phe
               660                 665                 670
   Asn Thr Arg Ala Thr Glu Glu Leu Leu Ala Phe Ala Lys Thr Ala Glu
           675                 680                 685
   Leu Ala Pro Gly Ala Ser Glu Thr Val Val Met His Phe Ser Ala Glu
       690                 695                 700
   Asp Leu Arg Arg Tyr Asp Gly Gln Arg Ser Ala Trp Val Val Asp Lys
   705                 710                 715                 720
   Gly Val Tyr Lys Leu Met Val Gly Ala Ser Ser Arg Asp Leu Arg Gly
                   725                 730                 735
   Phe Leu Ser Leu Asp Ile Pro His Glu Ile Val Val Leu Gln Thr Glu
               740                 745                 750
   Asn Leu Met Ala Pro Ala Glu Gln Ile Thr Glu Tyr Pro Val Asn Arg
           755                 760                 765
<210> 11
   <211> 1854
   <212> DNA
   <213> artificial sequence
<220>
   <223> arabinanase GH43 (Abn_43)
<400> 11
   agcccggaag gcgatgatcc cggccccgaa gaggcgaccg tttccatttc gacgccttcc      60
   gtaacgctcg ttacggaagg cggggcgaat gtggttgcaa ccgtcacgac caacaccccg     120
   tccgctattt taaagaaggg gttttgttac gacacccggt ccggcccgac gatctcgggt     180
   tcgaccgtcg agatggtcag cgcgggtttg tcgatcgatc tgacactgtc ggggctcgat     240
   cccgaaacga catacttcgt cagggcattc gccactgtgg tcggcaggga accggtctat     300
   tcacgagaga tcacattcac gacgacagcc aagggcgaga cttccgaact ggacgattac     360
   gtgccgcccg catatccgga taattacacc aatatcgcgg gctggagcga cagggccaaa     420
   tggaatctgg ccaacgtgca cgaccccagc gtggtattgg ccgaagacgg ctactattac     480
   atgtatcaga cggacgcttc gtacggcaat gcccacgaag ccggcggcca tttccacggt     540
   cgccgctcca aagaccttgt gaattgggag tacatgggag gcacgatgag tgcgttgccc     600
   gactgggtga tccccaagct gaacgagatc cgcacggaga tggggctgga tgaagtccac     660
   cccgatcccc ggaatttcgg ttactgggcc ccctgcgtcc ggaaggtcag ggacgggctc     720
   taccggatgt actattctat cgtctgcccg ggcctgatcg acggcgaagg gacctggagt     780
   gaacgcgcct tcatcggcct cgcggagaac accgaccccg ccgacaacga cgggtgggta     840
   gaccgcgggt atgtcgtcac gaactcttcg gatcgcgggt tggatttcca tgttcaggct     900
   aacgactggg cggactgtta ctacaaatgg acagccatcg acccgagcta catcatcgac     960
   accgacagca agcactggct ggtgtacggt tcgtggcaca gcggcatcgt cgcactggaa    1020
   atagacgcag cgacggggaa acccctcgac acactgcctg acccctgggg aacggaagaa    1080
   gatatcgctc cctatgggca attgctcgtg acacgccaaa tcgggaatcg ctggcaggga    1140
   tcagaagggc cggagatcgt atacaatccc gagaccggct attactatct gttcgtggct    1200
   tacgatgcgg tgggtgttcc ctataacact cgtgtgtgcc gttccacgaa catccttggc    1260
   ccgtacgtcg gcatcgacag ccgcgacctc acccggtatg gcggcgagat gttcccggtg    1320
   gtcacacatc cttacaagtt cgcgggcagc gacggctggg tgggtatttc gcactgcgct    1380
   atcttcgacg acgggaacgg gaactggttc tactcctcgc agggacgcct gcccgagaat    1440
   gtgccgggca taaacgccag caacgccgtt atgatgggac acgtgcgttc tatcctgtgg    1500
   acaagcaccg gctggccggt ggtcatgccc gaacgttacg gcgccgtacc caaagtgccc    1560
   atcacggaag acgaactgac gggaagctgg gagcacatag acctctccta ttcctacggg    1620
   agacagaaaa cattctccac catgacactt tccgccgatc acaaagtgac cgacggcgcc    1680
   tggaagggcg ctacgtggag ctatgacgcc gccaaccaac gcattgcgtt cagcaacggg    1740
   gtcgaagtgt gcctcgcgcg tgaagtggat tgggaagccg cgccgagaac ccactcgatc    1800
   gtctatgcgg gttacaccga aagcaaaaca tattggggga aaaaagaaaa atag 1854
<210> 12
   <211> 617
   <212> PRT
   <213> artificial sequence
<220>
   <223> arabinanase GH 43 (Abn_43)
<400> 12
   Ser Pro Glu Gly Asp Asp Pro Gly Pro Glu Glu Ala Thr Val Ser Ile
   1               5                   10                  15
   Ser Thr Pro Ser Val Thr Leu Val Thr Glu Gly Gly Ala Asn Val Val
               20                  25                  30
   Ala Thr Val Thr Thr Asn Thr Pro Ser Ala Ile Leu Lys Lys Gly Phe
           35                  40                  45
   Cys Tyr Asp Thr Arg Ser Gly Pro Thr Ile Ser Gly Ser Thr Val Glu
       50                  55                  60
   Met Val Ser Ala Gly Leu Ser Ile Asp Leu Thr Leu Ser Gly Leu Asp
   65                  70                  75                  80
   Pro Glu Thr Thr Tyr Phe Val Arg Ala Phe Ala Thr Val Val Gly Arg
                   85                  90                  95
   Glu Pro Val Tyr Ser Arg Glu Ile Thr Phe Thr Thr Thr Ala Lys Gly
               100                 105                 110
   Glu Thr Ser Glu Leu Asp Asp Tyr Val Pro Pro Ala Tyr Pro Asp Asn
           115                 120                 125
   Tyr Thr Asn Ile Ala Gly Trp Ser Asp Arg Ala Lys Trp Asn Leu Ala
       130                 135                 140
   Asn Val His Asp Pro Ser Val Val Leu Ala Glu Asp Gly Tyr Tyr Tyr
   145                 150                 155                 160
   Met Tyr Gln Thr Asp Ala Ser Tyr Gly Asn Ala His Glu Ala Gly Gly
                   165                 170                 175
   His Phe His Gly Arg Arg Ser Lys Asp Leu Val Asn Trp Glu Tyr Met
               180                 185                 190
   Gly Gly Thr Met Ser Ala Leu Pro Asp Trp Val Ile Pro Lys Leu Asn
           195                 200                 205
   Glu Ile Arg Thr Glu Met Gly Leu Asp Glu Val His Pro Asp Pro Arg
       210                 215                 220
   Asn Phe Gly Tyr Trp Ala Pro Cys Val Arg Lys Val Arg Asp Gly Leu
   225                 230                 235                 240
   Tyr Arg Met Tyr Tyr Ser Ile Val Cys Pro Gly Leu Ile Asp Gly Glu
                   245                 250                 255
   Gly Thr Trp Ser Glu Arg Ala Phe Ile Gly Leu Ala Glu Asn Thr Asp
               260                 265                 270
   Pro Ala Asp Asn Asp Gly Trp Val Asp Arg Gly Tyr Val Val Thr Asn
           275                 280                 285
   Ser Ser Asp Arg Gly Leu Asp Phe His Val Gln Ala Asn Asp Trp Ala
       290                 295                 300
   Asp Cys Tyr Tyr Lys Trp Thr Ala Ile Asp Pro Ser Tyr Ile Ile Asp
   305                 310                 315                 320
   Thr Asp Ser Lys His Trp Leu Val Tyr Gly Ser Trp His Ser Gly Ile
                   325                 330                 335
   Val Ala Leu Glu Ile Asp Ala Ala Thr Gly Lys Pro Leu Asp Thr Leu
               340                 345                 350
   Pro Asp Pro Trp Gly Thr Glu Glu Asp Ile Ala Pro Tyr Gly Gln Leu
           355                 360                 365
   Leu Val Thr Arg Gln Ile Gly Asn Arg Trp Gln Gly Ser Glu Gly Pro
       370                 375                 380
   Glu Ile Val Tyr Asn Pro Glu Thr Gly Tyr Tyr Tyr Leu Phe Val Ala
   385                 390                 395                 400
   Tyr Asp Ala Val Gly Val Pro Tyr Asn Thr Arg Val Cys Arg Ser Thr
                   405                 410                 415
   Asn Ile Leu Gly Pro Tyr Val Gly Ile Asp Ser Arg Asp Leu Thr Arg
               420                 425                 430
   Tyr Gly Gly Glu Met Phe Pro Val Val Thr His Pro Tyr Lys Phe Ala
           435                 440                 445
   Gly Ser Asp Gly Trp Val Gly Ile Ser His Cys Ala Ile Phe Asp Asp
       450                 455                 460
   Gly Asn Gly Asn Trp Phe Tyr Ser Ser Gln Gly Arg Leu Pro Glu Asn
   465                 470                 475                 480
   Val Pro Gly Ile Asn Ala Ser Asn Ala Val Met Met Gly His Val Arg
                   485                 490                 495
   Ser Ile Leu Trp Thr Ser Thr Gly Trp Pro Val Val Met Pro Glu Arg
               500                 505                 510
   Tyr Gly Ala Val Pro Lys Val Pro Ile Thr Glu Asp Glu Leu Thr Gly
           515                 520                 525
   Ser Trp Glu His Ile Asp Leu Ser Tyr Ser Tyr Gly Arg Gln Lys Thr
       530                 535                 540
   Phe Ser Thr Met Thr Leu Ser Ala Asp His Lys Val Thr Asp Gly Ala
   545                 550                 555                 560
   Trp Lys Gly Ala Thr Trp Ser Tyr Asp Ala Ala Asn Gln Arg Ile Ala
                   565                 570                 575
   Phe Ser Asn Gly Val Glu Val Cys Leu Ala Arg Glu Val Asp Trp Glu
               580                 585                 590
   Ala Ala Pro Arg Thr His Ser Ile Val Tyr Ala Gly Tyr Thr Glu Ser
           595                 600                 605
   Lys Thr Tyr Trp Gly Lys Lys Glu Lys
       610                 615
<210> 13
   <211> 924
   <212> DNA
   <213> artificial sequence
<220>
   <223> glucanase GH 8 (Gln_8)
<400> 13
   tgggaaacct ataaatcccg ctttgtgcag ccggacgggc gcgtggtgga taccggcaac      60
   ggcaacatct cgcacaccga agggcagggt tttgccatgc tgatggcggc gagcctcgac     120
   gatcgtcaga cgttcgataa gctctggcag tggaccaacg ccacgctgaa gaataaagac     180
   aacggcctgt tttactggcg ctacaatccg gtcgctccgg acccggtagc cgataaaaac     240
   gatgctaccg acggcgacat gatgatcgcc tgggcgctgc tgaaagcgca gaagcagtgg     300
   cgtgataaca gctacggtat ggcttccgat gaaatcacgc gcgcgttact caagcatacc     360
   gttatcagct atgcaggcta tcgggtgatg ctgcccggcg cgcatgggtt taacctgaac     420
   acccacatca acctcaatcc gtcttacttt atcttcccgg cctggcaggc ctttgcggac     480
   cgcagccacc tggtcgtctg gcgtgattta cagcgagacg gcaaaaagct gattggcgag     540
   atgggctggg gcaatgcgca attgccaacg gactgggtgg cgctcagcgc cgatggcaaa     600
   atgaaacctg ctgacgagtg gaaaccgcgc atgagctacg acgccattcg tattcccctc     660
   tatatccgct ggcaggagcc ggacagcccg ctgctcgcgc cgtggaaagc ctggtggcag     720
   cattaccaac gtgcgcaaac tccggcctgg gtgaacgtcc tgaacaatga aacctcgcca     780
   tacccgatga acggtggcct gttggccatc cgcgatttca ccttcggcga caatcccggc     840
   gagccgcaaa tcactccgca ggatgactac tactccgcca gcctgaagat gctgacctgg     900
   cttgccgggc gggatatcgc gaag                                            924
<210> 14
   <211> 308
   <212> PRT
   <213> artificial sequence
<220>
   <223> glucanase GH 8 (Gln_8)
<400> 14
   Trp Glu Thr Tyr Lys Ser Arg Phe Val Gln Pro Asp Gly Arg Val Val
   1 5 10 15
   Asp Thr Gly Asn Gly Asn Ile Ser His Thr Glu Gly Gln Gly Phe Ala
   20 25 30
   Met Leu Met Ala Ala Ser Leu Asp Asp Arg Gln Thr Phe Asp Lys Leu
   35 40 45
   Trp Gln Trp Thr Asn Ala Thr Leu Lys Asn Lys Asp Asn Gly Leu Phe
   50 55 60
   Tyr Trp Arg Tyr Asn Pro Val Ala Pro Asp Pro Val Ala Asp Lys Asn
   65 70 75 80
   Asp Ala Thr Asp Gly Asp Met Met Ile Ala Trp Ala Leu Leu Lys Ala
   85 90 95
   Gln Lys Gln Trp Arg Asp Asn Ser Tyr Gly Met Ala Ser Asp Glu Ile
   100 105 110
   Thr Arg Ala Leu Leu Lys His Thr Val Ile Ser Tyr Ala Gly Tyr Arg
   115 120 125
   Val Met Leu Pro Gly Ala His Gly Phe Asn Leu Asn Thr His Ile Asn
       130                 135                 140
   Leu Asn Pro Ser Tyr Phe Ile Phe Pro Ala Trp Gln Ala Phe Ala Asp
   145                 150                 155                 160
   Arg Ser His Leu Val Val Trp Arg Asp Leu Gln Arg Asp Gly Lys Lys
                   165                 170                 175
   Leu Ile Gly Glu Met Gly Trp Gly Asn Ala Gln Leu Pro Thr Asp Trp
               180                 185                 190
   Val Ala Leu Ser Ala Asp Gly Lys Met Lys Pro Ala Asp Glu Trp Lys
           195                 200                 205
   Pro Arg Met Ser Tyr Asp Ala Ile Arg Ile Pro Leu Tyr Ile Arg Trp
       210                 215                 220
   Gln Glu Pro Asp Ser Pro Leu Leu Ala Pro Trp Lys Ala Trp Trp Gln
   225                 230                 235                 240
   His Tyr Gln Arg Ala Gln Thr Pro Ala Trp Val Asn Val Leu Asn Asn
                   245                 250                 255
   Glu Thr Ser Pro Tyr Pro Met Asn Gly Gly Leu Leu Ala Ile Arg Asp
               260                 265                 270
   Phe Thr Phe Gly Asp Asn Pro Gly Glu Pro Gln Ile Thr Pro Gln Asp
           275                 280                 285
   Asp Tyr Tyr Ser Ala Ser Leu Lys Met Leu Thr Trp Leu Ala Gly Arg
       290                 295                 300
   Asp Ile Ala Lys
   305
<210> 15
   <211> 828
   <212> DNA
   <213> artificial sequence
<220>
   <223> estérase CE1 (CE_1-16)
<400> 15
   caattcaaca tgccgccgat gggccgcgtg gaaaaacaca ccatggagag caaggtactg 60
   ggcgtcgaaa gggactattc ggtctatctg cccaagagct tcgacaccga caaggataaa 120
   aaatatccca tcctgtacct gctccacggc atgctcgaca cccacaacgg atggcccgac      180
   agggggcatg tgggcgacgt ggccaaccag ctcaccgact ccggcgaaat ggtcgaaatg      240
   atcatcgtca cccccgacgc cggcggcaac atctgggaag gggcatggaa cggctatttc      300
   gacatgccgg gctggaagta cgaaaccttc ttcttcaccg agttcatgcc ctacatcgag      360
   gccaggtacc gcgtcatcgg cgacaaggag caccgcgccg tggccggcct ttcgatgggc      420
   ggcggcggag ccacttccta cggacagcag cattgggata aattctcgtc ggtctacgca      480
   atgagcgcgc tgatggacat caatgaggcc aacggagtgc ccgcccgcga gccggagaac      540
   cccgacgaca agatggcgct tctgacccgc tcggtcaagg aacacagctg catcggatat      600
   gtcgaggacg ccgacgacaa tcgcgtcgag cagttgcgtt cggtgaaatg gttcatcgat      660
   tgcggcgacg acgacttcct gctcgacgtc aacatcgacc tgacacgtgc catgcgcggg      720
   aaggccatac ctttcgagtt ccgcatccgt gacggcggcc acactttcga gtactggcat      780
   tcggcgcttt acatctgcct gccctacgtg tcgagaaatt tctcgaaa                   828
<210> 16
   <211> 276
   <212> PRT
   <213> artificial sequence
<220>
   <223> estérase CE1 (CE_1-16)
<400> 16
   Gln Phe Asn Met Pro Pro Met Gly Arg Val Glu Lys His Thr Met Glu
   1               5                   10                  15
   Ser Lys Val Leu Gly Val Glu Arg Asp Tyr Ser Val Tyr Leu Pro Lys
               20                  25                  30
   Ser Phe Asp Thr Asp Lys Asp Lys Lys Tyr Pro Ile Leu Tyr Leu Leu
           35                  40                  45
   His Gly Met Leu Asp Thr His Asn Gly Trp Pro Asp Arg Gly His Val
       50                  55                  60
   Gly Asp Val Ala Asn Gln Leu Thr Asp Ser Gly Glu Met Val Glu Met
   65                  70                  75                  80
   Ile Ile Val Thr Pro Asp Ala Gly Gly Asn Ile Trp Glu Gly Ala Trp
                   85                  90                  95
   Asn Gly Tyr Phe Asp Met Pro Gly Trp Lys Tyr Glu Thr Phe Phe Phe
               100                 105                 110
   Thr Glu Phe Met Pro Tyr Ile Glu Ala Arg Tyr Arg Val Ile Gly Asp
           115                 120                 125
   Lys Glu His Arg Ala Val Ala Gly Leu Ser Met Gly Gly Gly Gly Ala
       130                 135                 140
   Thr Ser Tyr Gly Gln Gln His Trp Asp Lys Phe Ser Ser Val Tyr Ala
   145                 150                 155                 160
   Met Ser Ala Leu Met Asp Ile Asn Glu Ala Asn Gly Val Pro Ala Arg
                   165                 170                 175
   Glu Pro Glu Asn Pro Asp Asp Lys Met Ala Leu Leu Thr Arg Ser Val
               180                 185                 190
   Lys Glu His Ser Cys Ile Gly Tyr Val Glu Asp Ala Asp Asp Asn Arg
           195                 200                 205
   Val Glu Gln Leu Arg Ser Val Lys Trp Phe Ile Asp Cys Gly Asp Asp
       210                 215                 220
   Asp Phe Leu Leu Asp Val Asn Ile Asp Leu Thr Arg Ala Met Arg Gly
   225                 230                 235                 240
   Lys Ala Ile Pro Phe Glu Phe Arg Ile Arg Asp Gly Gly His Thr Phe
                   245                 250                 255
   Glu Tyr Trp His Ser Ala Leu Tyr Ile Cys Leu Pro Tyr Val Ser Arg
               260                 265                 270
   Asn Phe Ser Lys
           275
<210> 17
   <211> 795
   <212> DNA
   <213> artificial sequence
<220>
   <223> estérase CE1 (CE_1-18)
<400> 17
   agctatgtga tcgttccggg cgagcgcacc gcaggttacg aagagtacag cggcaacaag        60
   ggcaccgttt cgaaggtatg gtaccactcg ccggcttacg gcgccgaccg ccgcatgtac       120
   atctacactc cttacggtta cgaaggcggc aaagcgaaat atccggtgtt gtatctgcag       180
   cacggaggcg gaggcgacga agatgcatgg acttcgctgg gtcgtgcggc gcagatcctc       240
   gaccggttga tcggcgaagg caaggccgtg ccgatgatcg tggtgatgcc caatgccaac       300
   ccgaaccagc tggccgctcc cgacgtgatg gccccgatgc ccgtcgagcg cattccgcaa       360
   ggcgacccgt cgttccaccg cgggggtaaa tacgtcgaaa gtttcagcgc cgacatcgtt       420
   ccgttcgtcg attcgcatta ccgcacgatc gctaagaaga gcggccgtgc cattgccggg       480
   ctttcgatgg gaggcatcta tactctgtcg ataaccgccg cgcatccgga gctgtgtgac       540
   tacatcggcg tgctgagcat gggtttcacc cccgacaaag accccgaaac ggaactcggc       600
   cccatcaaga aagcgggcta caagctctac tgggtgggtt gtggcgaatc cgattttgca       660
   tacggcaacg cggaaaggct gctggaaggg ctcgacaaga tgaacatgcc acacgaattc       720
   ttcaacgaga tcggcgggca cacctgggcc acatggagga tctgcctgga cgaactcgca       780
   cctaaactgt tcaaa                                                        795
<210> 18
   <211> 265
   <212> PRT
   <213> artificial sequence
<220>
   <223> estérase CE1 (CE_1-18)
<400> 18
   Ser Tyr Val Ile Val Pro Gly Glu Arg Thr Ala Gly Tyr Glu Glu Tyr
   1               5                   10                  15
   Ser Gly Asn Lys Gly Thr Val Ser Lys Val Trp Tyr His Ser Pro Ala
               20                  25                  30
   Tyr Gly Ala Asp Arg Arg Met Tyr Ile Tyr Thr Pro Tyr Gly Tyr Glu
           35                  40                  45
   Gly Gly Lys Ala Lys Tyr Pro Val Leu Tyr Leu Gln His Gly Gly Gly
       50                  55                  60
   Gly Asp Glu Asp Ala Trp Thr Ser Leu Gly Arg Ala Ala Gln Ile Leu
   65                  70                  75                  80
   Asp Arg Leu Ile Gly Glu Gly Lys Ala Val Pro Met Ile Val Val Met
                   85                  90                  95
   Pro Asn Ala Asn Pro Asn Gln Leu Ala Ala Pro Asp Val Met Ala Pro
               100                 105                 110
   Met Pro Val Glu Arg Ile Pro Gln Gly Asp Pro Ser Phe His Arg Gly
           115                 120                 125
   Gly Lys Tyr Val Glu Ser Phe Ser Ala Asp Ile Val Pro Phe Val Asp
       130                 135                 140
   Ser His Tyr Arg Thr Ile Ala Lys Lys Ser Gly Arg Ala Ile Ala Gly
   145                 150                 155                 160
   Leu Ser Met Gly Gly Ile Tyr Thr Leu Ser Ile Thr Ala Ala His Pro
                   165                 170                 175
   Glu Leu Cys Asp Tyr Ile Gly Val Leu Ser Met Gly Phe Thr Pro Asp
               180                 185                 190
   Lys Asp Pro Glu Thr Glu Leu Gly Pro Ile Lys Lys Ala Gly Tyr Lys
           195                 200                 205
   Leu Tyr Trp Val Gly Cys Gly Glu Ser Asp Phe Ala Tyr Gly Asn Ala
       210                 215                 220
   Glu Arg Leu Leu Glu Gly Leu Asp Lys Met Asn Met Pro His Glu Phe
   225                 230                 235                 240
   Phe Asn Glu Ile Gly Gly His Thr Trp Ala Thr Trp Arg Ile Cys Leu
                   245                 250                 255
   Asp Glu Leu Ala Pro Lys Leu Phe Lys
               260                 265
<210> 19
   <211> 1047
   <212> DNA
   <213> artificial sequence
<220>
   <223> Glucanase GH8 (Gln_8-20)
<400> 19
   gcggcctgcg actggcctga gtggacgcag tttaaaaagg attacatcag cgaagaggga      60
   cgggtgattg atccgagcga cgcgcggaaa atcaccacct ccgaggggca aagctatgcg     120
   ctgttctttg cccttgcggc gaacgatcgt gaggccttcg cttcattgtt caactggacg     180
   caaaacaatc tggctgaagg ggatttaaag gcgcatcttc ctgcctggct gtggggcaaa     240
   aaaggggatg atgactggaa agtgctcgac agcaattcgg cctcggattc cgatatctgg     300
   atagcctggt cgctactgga agccgggcgt ctgtggaaca tgccgcagta cagtgagacc     360
   gggaaagccc tgctgtcacg catcgccaaa gaagaagtgg tgaaggtgcc agggcttggc     420
   tccatgcttc tgccgggtaa agtgggtttt gcggatgagg ccgggtggcg ctttaacccg     480
   agctatttgc ctccgcaact ggctgcctac tttgcccgct acggggtacc gtgggatgca     540
   cttcaggcaa ccaatttgcg attgctgctg gaaaccgcgc ctaagggcta ttcgccaaac     600
   tgggtgcgct tcgacaagaa aaaaggctgg cagctgaagc aggacaagac gcttattggc      660
   agctatgacg cgattcgggt ctacctgtgg gtggggatgc tcaacgatga cgatccgcaa      720
   aaagcgcggc tattgaagaa attcgccccg atggcggcac aaaccaccaa agcgggcgtt      780
   gtgccagaga aagtggcgat cgccagcggt aaagtggagg gggatggccc ggttggattc      840
   tccgctgcgc tgctgccgtt cttacaaaat cgcgatgctc aggccgttca gcgccagcgc      900
   gtcgccgacc atttcccagg caaagatacc tattacagct atgttctgac cctgttcggg      960
   caaggatggg atcaacaccg ttttcgcttc accgctcgcg gcgagctttt acctgactgg     1020
   ggccaggaat gcgtaagttc acactaa                                         1047
<210> 20
   <211> 348
   <212> PRT
   <213> artificial sequence
<220>
   <223> Glucanase GH8 (Gln_8-20)
<400> 20
   Ala Ala Cys Asp Trp Pro Glu Trp Thr Gln Phe Lys Lys Asp Tyr Ile
   1               5                   10                  15
   Ser Glu Glu Gly Arg Val Ile Asp Pro Ser Asp Ala Arg Lys Ile Thr
               20                  25                  30
   Thr Ser Glu Gly Gln Ser Tyr Ala Leu Phe Phe Ala Leu Ala Ala Asn
           35                  40                  45
   Asp Arg Glu Ala Phe Ala Ser Leu Phe Asn Trp Thr Gln Asn Asn Leu
       50                  55                  60
   Ala Glu Gly Asp Leu Lys Ala His Leu Pro Ala Trp Leu Trp Gly Lys
   65                  70                  75                  80
   Lys Gly Asp Asp Asp Trp Lys Val Leu Asp Ser Asn Ser Ala Ser Asp
                   85                  90                  95
   Ser Asp Ile Trp Ile Ala Trp Ser Leu Leu Glu Ala Gly Arg Leu Trp
               100                 105                 110
   Asn Met Pro Gln Tyr Ser Glu Thr Gly Lys Ala Leu Leu Ser Arg Ile
           115                 120                 125
   Ala Lys Glu Glu Val Val Lys Val Pro Gly Leu Gly Ser Met Leu Leu
       130                 135                 140
   Pro Gly Lys Val Gly Phe Ala Asp Glu Ala Gly Trp Arg Phe Asn Pro
   145 150 155 160
   Ser Tyr Leu Pro Pro Gln Leu Ala Ala Tyr Phe Ala Arg Tyr Gly Val
   165 170 175
   Pro Trp Asp Ala Leu Gln Ala Thr Asn Leu Arg Leu Leu Leu Glu Thr
   180 185 190
   Ala Pro Lys Gly Tyr Ser Pro Asn Trp Val Arg Phe Asp Lys Lys Lys
   195 200 205
   Gly Trp Gln Leu Lys Gln Asp Lys Thr Leu Ile Gly Ser Tyr Asp Ala
   210 215 220
   Ile Arg Val Tyr Leu Trp Val Gly Met Leu Asn Asp Asp Asp Pro Gln
   225 230 235 240
   Lys Ala Arg Leu Leu Lys Lys Phe Ala Pro Met Ala Ala Gln Thr Thr
   245 250 255
   Lys Ala Gly Val Val Pro Glu Lys Val Ala Ile Ala Ser Gly Lys Val
   260 265 270
   Glu Gly Asp Gly Pro Val Gly Phe Ser Ala Ala Leu Leu Pro Phe Leu
   275 280 285
   Gln Asn Arg Asp Ala Gln Ala Val Gln Arg Gln Arg Val Ala Asp His
   290 295 300
   Phe Pro Gly Lys Asp Thr Tyr Tyr Ser Tyr Val Leu Thr Leu Phe Gly
   305 310 315 320
   Gln Gly Trp Asp Gln His Arg Phe Arg Phe Thr Ala Arg Gly Glu Leu
   325 330 335
   Leu Pro Asp Trp Gly Gln Glu Cys Val Ser Ser His
   340 345
<210> 21
   <211> 1500
   <212> DNA
   <213> artificial sequence
<220>
   <223> Arabinofuranosidase GH51 (Abf_51)
<400> 21
   ttccgagcac acctcactgt cgatccgcac ttcacggtcg gcccgatcaa cagacgcctc 60
   ttcggctcgt tcgtcgagca ccttggccga tgcgtctacg acggcatcta cgagccatcg 120
   cacgcgacag ccgatgagca cggcttccgc tccgatgtcg tcgaactcgt gcgtgagctg 180
   ggagtctcga cgatcaggta tccgggcggg aacttcgtct ccggctaccg atgggaggac 240
   ggcatcgggc cgcgtgacga gcggccccgg cgactcgacc tcgcctggca ttcgaccgag 300
   acgaacgagg tcggtctcga cgagttcgcg acctggctcg gcaaggtgga cagcgagctc 360
   atgtacgcgg tgaacctcgg gacgcgcgga gtgctcgagg cgctcgatgt gctggagtac 420
   gccaacctgc cggccggcac ccattgggcc gatcggcgcg ttgcgaacgg acacgccgag 480
   cctcacggca tccgcatgtg gtgcctcggc aacgagatgg acggcccttg gcagctcggc 540
   cacagcacgg cggaggagta tgcccagctt gcggcgacga ccgcggcggc catgcgccag 600
   atcgaccccg acctcgagct cgtggtgtgc ggcagctcgg gggcgcagat gcccaccttc 660
   ggcgagtggg agcgcatcgt gctcgagaag acgtacgaca gcgtcgactt catctcgtgc 720
   cacgcctact acgagccgaa gggcgacgac tacggcagct tcctcgcgtc ggcggtcaac 780
   atggaccgct tcatcgatgc cgtcgtcgcc acggcggatt cggtgaaggc gcgcctgcgc 840
   agcgacaagt cgatcaacat ctcgttcgac gagtggaacg tctggtacca gacgcgctat 900
   caggaggtcg accggatcac ggacgtcaac agctggccgg tcgccccgcg tctgctcgag 960
   gactcgtact cggtgctcga cgccgttgtc ttcggcagcc tcatgatctc gctcatccgc 1020
   cacgcagacc gcgtcacggc ggcgagcctc gcccagctcg tgaacgtcat cgccccgatc 1080
   atgaccgagc ccggcggccc ggcatggaag cagacgactt tccacccgtt ctcgctcacg 1140
   tcccggctcg ctcgcggcac ggcactcgag ctcaagctgg acagcccgac ctaccagacg 1200
   caggagtacg gcgaggtgag ccttgtcgac gcggtcgcga cgcatgacga ggggggcacg 1260
   acggtcttcg cggtcaaccg gagtctcacc gacgaagcga ccctcgagat cgacacgcgt 1320
   gcgctcggcg cggtcgcgca ggcggaggcg acatccctcc atgacgacga catccatgcg 1380
   gccaacacgc tcgacgatcg cgagcgtgtg agcccgcggc cgaacgaatc cgtgcagatc 1440
   ggcgacggcg tcgtcaccat cacgctccct ccggtgtcgt ggacggtgct gacgctcgcc 1500
<210> 22
   <211> 500
   <212> PRT
   <213> artificial sequence
<220>
   <223> Arabinofuranosidase GH51 (Abf_51)
<400> 22
   Phe Arg Ala His Leu Thr Val Asp Pro His Phe Thr Val Gly Pro Ile
   1               5                   10                  15
   Asn Arg Arg Leu Phe Gly Ser Phe Val Glu His Leu Gly Arg Cys Val
               20                  25                  30
   Tyr Asp Gly Ile Tyr Glu Pro Ser His Ala Thr Ala Asp Glu His Gly
           35                  40                  45
   Phe Arg Ser Asp Val Val Glu Leu Val Arg Glu Leu Gly Val Ser Thr
       50                  55                  60
   Ile Arg Tyr Pro Gly Gly Asn Phe Val Ser Gly Tyr Arg Trp Glu Asp
   65                  70                  75                  80
   Gly Ile Gly Pro Arg Asp Glu Arg Pro Arg Arg Leu Asp Leu Ala Trp
                   85                  90                  95
   His Ser Thr Glu Thr Asn Glu Val Gly Leu Asp Glu Phe Ala Thr Trp
               100                 105                 110
   Leu Gly Lys Val Asp Ser Glu Leu Met Tyr Ala Val Asn Leu Gly Thr
           115                 120                 125
   Arg Gly Val Leu Glu Ala Leu Asp Val Leu Glu Tyr Ala Asn Leu Pro
       130                 135                 140
   Ala Gly Thr His Trp Ala Asp Arg Arg Val Ala Asn Gly His Ala Glu
   145                 150                 155                 160
   Pro His Gly Ile Arg Met Trp Cys Leu Gly Asn Glu Met Asp Gly Pro
                   165                 170                 175
   Trp Gln Leu Gly His Ser Thr Ala Glu Glu Tyr Ala Gln Leu Ala Ala
               180                 185                 190
   Thr Thr Ala Ala Ala Met Arg Gln Ile Asp Pro Asp Leu Glu Leu Val
           195                 200                 205
   Val Cys Gly Ser Ser Gly Ala Gln Met Pro Thr Phe Gly Glu Trp Glu
       210                 215                 220
   Arg Ile Val Leu Glu Lys Thr Tyr Asp Ser Val Asp Phe Ile Ser Cys
   225                 230                 235                 240
   His Ala Tyr Tyr Glu Pro Lys Gly Asp Asp Tyr Gly Ser Phe Leu Ala
                   245                 250                 255
   Ser Ala Val Asn Met Asp Arg Phe Ile Asp Ala Val Val Ala Thr Ala
               260                 265                 270
   Asp Ser Val Lys Ala Arg Leu Arg Ser Asp Lys Ser Ile Asn Ile Ser
   275 280 285
   Phe Asp Glu Trp Asn Val Trp Tyr Gln Thr Arg Tyr Gln Glu Val Asp
   290 295 300
   Arg Ile Thr Asp Val Asn Ser Trp Pro Val Ala Pro Arg Leu Leu Glu
   305 310 315 320
   Asp Ser Tyr Ser Val Leu Asp Ala Val Val Phe Gly Ser Leu Met Ile
   325 330 335
   Ser Leu Ile Arg His Ala Asp Arg Val Thr Ala Ala Ser Leu Ala Gln
   340 345 350
   Leu Val Asn Val Ile Ala Pro Ile Met Thr Glu Pro Gly Gly Pro Ala
   355 360 365
   Trp Lys Gln Thr Thr Phe His Pro Phe Ser Leu Thr Ser Arg Leu Ala
   370 375 380
   Arg Gly Thr Ala Leu Glu Leu Lys Leu Asp Ser Pro Thr Tyr Gln Thr
   385 390 395 400
   Gln Glu Tyr Gly Glu Val Ser Leu Val Asp Ala Val Ala Thr His Asp
   405 410 415
   Glu Gly Gly Thr Thr Val Phe Ala Val Asn Arg Ser Leu Thr Asp Glu
   420 425 430
   Ala Thr Leu Glu Ile Asp Thr Arg Ala Leu Gly Ala Val Ala Gln Ala
   435 440 445
   Glu Ala Thr Ser Leu His Asp Asp Asp Ile His Ala Ala Asn Thr Leu
   450 455 460
   Asp Asp Arg Glu Arg Val Ser Pro Arg Pro Asn Glu Ser Val Gln Ile
   465 470 475 480
   Gly Asp Gly Val Val Thr Ile Thr Leu Pro Pro Val Ser Trp Thr Val
   485 490 495
   Leu Thr Leu Ala
   500
<210> 23
   <211> 76
   <212> DNA
   <213> artificial sequence
<220>
   <223> forward primer Xyn_10
<400> 23
   ggggacaagt ttgtacaaaa aagcaggctt agaaaacctg tacttccagg gtgaaaaaat 60
   gaagtgggac gatccc 76
<210> 24
   <211> 57
   <212> DNA
   <213> artificial sequence
<220>
   <223> reverse primer Xyn_10
<400> 24
   ggggaccact ttgtacaaga aagctgggtc ttattacttt gctgcgtcgt tcacaag 57
<210> 25
   <211> 73
   <212> DNA
   <213> artificial sequence
<220>
   <223> forward primer Xyl_3
<400> 25
   ggggacaagt ttgtacaaaa aagcaggctt agaaaacctg tacttccagg gtcaggaagc 60
   gccccaactt cgc 73
<210> 26
   <211> 59
   <212> DNA
   <213> artificial sequence
<220>
   <223> reverse primer Xyl_3
<400> 26
   ggggaccact ttgtacaaga aagctgggtc ttattatgtg cgaacgatca gttccagca 59
<210> 27
   <211> 73
   <212> DNA
   <213> artificial sequence
<220>
   <223> forward primer Abf_97
<400> 27
   ggggacaagt ttgtacaaaa aagcaggctt agaaaacctg tacttccagg gtcatccggc 60
   ggacgacttc tcc 73
<210> 28
   <211> 58
   <212> DNA
   <213> artificial sequence
<220>
   <223> reverse primer Abf_97
<400> 28
   ggggaccact ttgtacaaga aagctgggtc ttattatttt gtggtgatag ttatcccg 58
<210> 29
   <211> 74
   <212> DNA
   <213> artificial sequence
<220>
   <223> forward primer Abf_51
<400> 29
   ggggacaagt ttgtacaaaa aagcaggctt agaaaacctg tacttccagg gtttccgagc 60
   acacctcact gtcg 74
<210> 30
   <211> 54
   <212> DNA
   <213> artificial sequence
<220>
   <223> reverse primer Abf_51
<400> 30
   ggggaccact ttgtacaaga aagctgggtc tcatcaggcg agcgtcagca ccgt 54
<210> 31
   <211> 73
   <212> DNA
   <213> artificial sequence
<220>
   <223> forward primer Abn_43
<400> 31
   ggggacaagt ttgtacaaaa aagcaggctt agaaaacctg tacttccagg gtagcccgga 60
   aggcgatgat ccc 73
<210> 32
   <211> 61
   <212> DNA
   <213> artificial sequence
<220>
   <223> reverse primer Abn_43
<400> 32
   ggggaccact ttgtacaaga aagctgggtc ttattactat ttttcttttt tcccccaata 60
   t 61
<210> 33
   <211> 76
   <212> DNA
   <213> artificial sequence
<220>
   <223> forward primer Gln_8
<400> 33
   ggggacaagt ttgtacaaaa aagcaggctt agaaaacctg tacttccagg gttgggaaac 60
   ctataaatcc cgcttt 76
<210> 34
   <211> 54
   <212> DNA
   <213> artificial sequence
<220>
   <223> reverse primer Gln_8
<400> 34
   ggggaccact ttgtacaaga aagctgggtc ctactacttc gcgatatccc gccc 54
<210> 35
   <211> 72
   <212> DNA
   <213> artificial sequence
<220>
   <223> forward primer Gln_20
<400> 35
   ggggacaagt ttgtacaaaa aagcaggctt agaaaacctg tacttccagg gtgcggcctg 60
   cgactggcct ga 72
<210> 36
   <211> 57
   <212> DNA
   <213> artificial sequence
<220>
   <223> reverse primer Gln_8-20
<400> 36
   ggggaccact ttgtacaaga aagctgggtc ttattagtgt gaacttacgc attcctg 57
<210> 37
   <211> 74
   <212> DNA
   <213> artificial sequence
<220>
   <223> forward primer CE_1-16
<400> 37
   ggggacaagt ttgtacaaaa aagcaggctt agaaaacctg tacttccagg gtcaattcaa 60
   catgccgccg atgg 74
<210> 38
   <211> 58
   <212> DNA
   <213> artificial sequence
<220>
   <223> reverse primer CE_1-16
<400> 38
   ggggaccact ttgtacaaga aagctgggtc ttattatttc gagaaatttc tcgacacg 58
<210> 39
   <211> 74
   <212> DNA
   <213> artificial sequence
<220>
   <223> forward primer CE_1-18
<400> 39
   ggggacaagt ttgtacaaaa aagcaggctt agaaaacctg tacttccagg gtagctatgt 60
   gatcgttccg ggcg 74
<210> 40
   <211> 58
   <212> DNA
   <213> artificial sequence
<220>
   <223> reverse primer CE_1-18
<400> 40
   ggggaccact ttgtacaaga aagctgggtc tcatcatttg aacagtttag gtgcgagt 58